# EUROPÄISCHE PATENTSCHRIFT

(11) **EP 0 589 322 B1**
(45) Veröffentlichungstag und Bekanntmachung des Hinweises auf die Patenterteilung: **19.03.1997**
(21) Anmeldenummer: 93114676.5
(22) Anmeldetag: 13.09.1993
(51) Int. Cl.: C07C 271/20, A61K 31/27, C07D 295/13, C07C 317/44, C07D 215/48, C07D 209/42

(54) **5-Oxo-dibenzo[a,d]cyclohepta-1,4-diene und ihre Verwendung als retrovirale Mittel**
5-Oxo-dibenzo[a,d]cyclohepta-1,4-dienes and their use as retroviral agents
5-Oxo-dibenzo[a,d]cyclohepta-1,4-diènes et leur utilisation comme agents rétroviraux

(30) Priorität: 25.09.1992 DE 4232173
(43) Veröffentlichungstag der Anmeldung: 30.03.1994
(73) Patentinhaber: BAYER AG, 51368 Leverkusen (DE)
(72) Erfinder: Wild, Hanno, Dr., D-42113 Wuppertal (DE); Hansen, Jutta, Dr., D-42115 Wuppertal (DE); Lautz, Jörg, Dr., D-42489 Wülfrath (DE); Paessens, Arnold, Dr., D-42781 Haan (DE)

(56) Entgegenhaltungen:
- US-A- 3 436 402
- BULLETIN DE LA SOCIETE CHIMIQUE DE FRANCE 1960 , PARIS FR Seiten 400 - 405 J. RIGAUDY, L NEDELEC 'Obtention et réarrangement de l'oxyde de méthylène-anthrone, de l'oxyde de dibenzo-2,3,6,7 tropone et des diols correspondants, les dihydroxy-4,5 dibenzo-2,3-6,7 cycloheptadienones-I'

## Beschreibung

Die Erfindung betrifft 5-Oxo-dibenzo[a,d]cyclohepta-1,4-diene, Verfahren zu ihrer Herstellung und ihre Verwendung als retrovirale Mittel.

Cis/trans-10,11-Dihydroxy-5-oxo-dibenzo[a,d]cyclohepta-1,4-dien und die entsprechenden Diacetate sind aus der Publikation Bull. Soc. Chim. Fr. (1960), 400 bereits bekannt.

Die vorliegende Erfindung betrifft jetzt substituierte 5-Oxo-dibenzo[a,d]cyclohepta-1,4-diene der allgemeinen Formel (I) 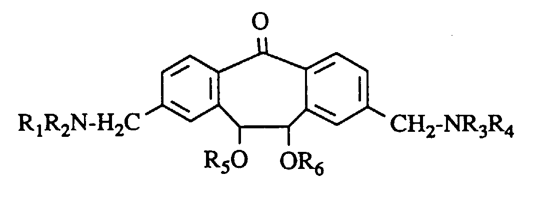 in welcher
- R¹ und R³: gleich oder verschieden sind und für eine Aminoschutzgruppe stehen oder für eine Gruppe der Formel R⁷-CO- stehen,
worin
- R⁷: Wasserstoff, Trifluormethyl oder geradkettiges oder verzweigtes Alkoxy mit bis zu 8 Kohlenstoffatomen oder Alkyl mit bis zu 18 Kohlenstoffatomen bedeutet, die gegebenenfalls bis zu 2-fach gleich oder verschieden durch Aryl mit 6 bis 10 Kohlenstoffatomen oder Pyridyl substituiert sind, oder
Aryl mit 6 bis 10 Kohlenstoffatomen bedeutet, das gegebenenfalls durch Halogen, Trifluormethyl, Trifluormethoxy oder durch geradkettiges oder verzweigtes Alkyl mit bis zu 8 Kohlenstoffatomen substituiert ist,
Cycloalkyl mit 3 bis 7 Kohlenstoffatomen bedeutet, oder
Chinolyl, Chinolyl-N-oxid, Indolyl, Pyridyl, Morpholino oder Piperazinyl bedeutet, oder
einen Rest der Formel

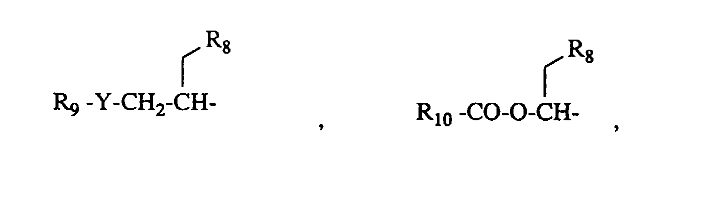

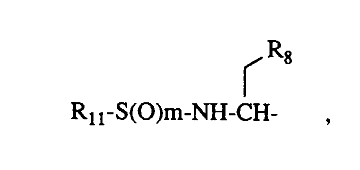

T-NH-(CH₂)ₚ- ,

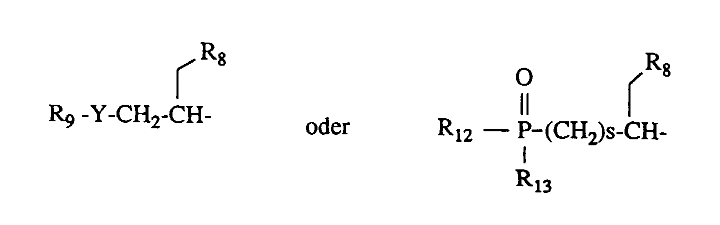

bedeutet,
worin
R⁸ Phenyl oder Naphthyl bedeutet,
R⁹, R¹⁰ und R¹¹ unabhängig voneinander geradkettiges oder verzweigtes Alkyl mit bis zu 14 Kohlenstoffatomen bedeuten, das gegebenenfalls durch Phenyl oder Naphthyl substituiert ist, oder Aryl mit 6 bis 10 Kohlenstoffatomen bedeuten, welches seinerseits durch Alkyl mit bis zu 4 Kohlenstoffatomen substituiert ist, oder
R¹⁰ einen Rest der Formel 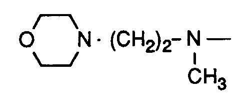 bedeutet,
m eine Zahl 0, 1 oder 2 bedeutet,
T Morpholino oder Cyclohexyl bedeutet,
p eine Zahl 1, 2 oder 3 bedeutet,
Y und Y' unabhängig voneinander für CO- oder SO₂- stehen,
t eine Zahl 0 oder 1 bedeutet,
R¹² und R¹³ unabhängig voneinander für Hydroxy oder Alkoxy mit bis zu 8 Kohlenstoffatomen stehen,
s für eine Zahl 1 oder 2 steht,
- R² und R⁴: gleich oder verschieden sind und für Wasserstoff oder geradkettiges oder verzweigtes Alkyl mit bis zu 8 Kohlenstoffatomen stehen,
- R⁵ und R⁶: gleich oder verschieden sind und für Wasserstoff oder für geradkettiges oder verzweigtes Acyl oder Alkoxycarbonyl mit jeweils bis zu 8 Kohlenstoffatomen oder für eine Hydroxyschutzgruppe stehen,
und im Fall, daß entweder R⁵ oder R⁶ für Wasserstoff steht,
in Form ihrer Halbacetale und deren Salze.

Physiologisch unbedenkliche Salze der substituierten 5-Oxo-dibenzo[a,d]cyclohepta-1,4-diene können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methyl-piperidin.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Aminoschutzgruppe im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.Butoxycarbonyl, Allyloxycarbonyl, Vinyloxycarbonyl, 2-Nitrobenzyloxycarbonyl, 3,4,5-Trimethoxybenzyloxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tertbutoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl.

Bei dem Rest der Formel (II) 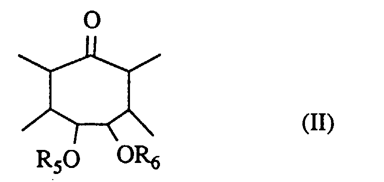 können die Gruppen -OR⁵ und -OR⁶ in cis- oder trans-Stellung zueinander stehen oder als cis/trans-Isomerengemisch vorliegen.

Die Definition der Halbacetalform wird durch den Rest der Formel (IIa) beispielhaft erläutert: 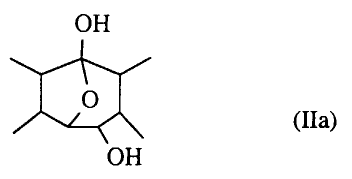 Hydroxyschutzgruppe steht im allgemeinen für Trimethylsilyl, Triethylsilyl, Triisopropylsilyl, tert.Butyldimethylsilyl, Triphenylsilyl, Trimethylsilylethoxycarbonyl, Benzyl, Benzyloxycarbonyl, 2-Nitrobenzyl, 4-Nitrobenzyl, 2-Nitrobenzyloxycarbonyl, 4-Nitrobenzyloxycarbonyl, tert.Butoxycarbonyl, Allyloxycarbonyl, 4-Methoxybenzyloxycarbonyl, Formyl, Acetyl, Trichloracetyl, 2,2,2-Trichlorethoxycarbonyl, 2,4-Dimethoxycarbonyl, 2,4-Dimethoxybenzyloxycarbonyl, Methoxymethyl, Methylthiomethyl, Methoxyethoxymethyl, [2-(Trimethylsilyl)ethoxy]methyl, 2-(Methylthiomethoxy)ethoxycarbonyl, Tetrahydropyranyl oder Benzoyl.
Bevorzugt sind Trimethylsilyl, Triethylsilyl, Triisopropylsilyl, tert.Butyl-dimethylsilyl, Triphenylsilyl, Benzyloxycarbonyl, 4-Nitrobenzyloxycarbonyl, tert.Butoxycarbonyl, Allyloxycarbonyl, 4-methoxybenzyloxycarbonyl, Formyl, Acetyl, Trichloracetyl.

Bevorzugt sind Verbindungen der allgemeinen Formel (I),
in welcher
- R¹ und R³: gleich oder verschieden sind und für Benzyloxycarbonyl, 4-Methoxybenzyloxycarbonyl, 4-Nitrobenzyloxycarbonyl, tert.Butoxycarbonyl, Allyloxycarbonyl, 2-Nitrobenzyloxycarbonyl, Fluorenyl-9-methoxycarbonyl oder 2,2,2-Trifluoracetyl stehen, oder
für eine Gruppe der Formel R⁷-CO- stehen,
R⁷ Wasserstoff, Trifluormethyl oder geradkettiges oder verzweigtes Alkoxy mit bis zu 4 Kohlenstoffatomen oder Alkyl mit bis zu 16 Kohlenstoffatomen bedeutet, die gegebenenfalls bis zu 2-fach, gleich oder verschieden, durch Phenyl, Naphthyl oder Pyridyl substituiert sind, oder
Phenyl oder Naphthyl bedeutet, die gegebenenfalls durch Fluor, Chlor, Trifluormethyl, Trifluormethoxy oder durch geradkettiges oder verzweigtes Alkyl mit bis zu 6 Kohlenstoffatomen substituiert sein können,
Cyclopropyl, Cyclopentyl, Cyclohexyl, Chinolyl, Chinolyl-N-oxid, Indolyl, Pyridyl, Morpholino oder Piperazinyl bedeutet, oder
einen Rest der Formel 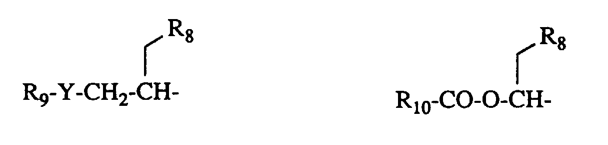 oder 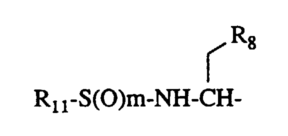 bedeutet, worin
Y die CO- oder SO₂-Gruppe bedeutet,
R⁸ Phenyl oder Naphthyl bedeutet,
R⁹, R¹⁰ und R¹¹ unabhängig voneinander für geradkettiges oder verzweigtes Alkyl mit bis zu 8 Kohlenstoffatomen, Tolyl, Phenyl oder Naphthyl stehen, oder
R¹⁰ einen Rest der Formel 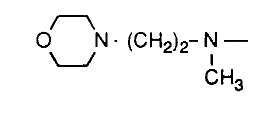 bedeutet,
m eine Zahl 1 oder 2 bedeutet,
- R² und R⁴: gleich oder verschieden sind und für Wasserstoff oder geradkettiges oder verzweigtes Alkyl mit bis zu 6 Kohlenstoffatomen stehen,
- R⁵ und R⁶: gleich oder verschieden sind und für Wasserstoff oder für geradkettiges oder verzweigtes Acyl oder Alkoxycarbonyl mit jeweils bis zu 6 Kohlenstoffatomen oder
für Trimethylsilyl, Triethylsilyl, Triisopropylsilyl, tert.Butyl-dimethylsilyl, Triphenylsilyl, Benzyloxycarbonyl, 4-Nitrobenzyloxycarbonyl, tert.Butoxycarbonyl, Allyloxycarbonyl, 4-Methoxybenzyloxycarbonyl, Formyl, Acetyl oder Trichloracetyl stehen,
und im Fall, daß entweder R⁵ oder R⁶ für Wasserstoff steht,
in Form ihrer Halbacetale und deren Salze.

Besonders bevorzugt sind Verbindungen der allgemeinen Formel (I),
in welcher
- R¹ und R³: gleich oder verschieden sind und für Benzyloxycarbonyl, 4-Methoxybenzyloxycarbonyl, 4-Nitrobenzyloxycarbonyl, tert.Butoxycarbonyl, oxycarbonyl, 2-Nitrobenzyloxycarbonyl, Fluorenyl-9-methoxycarbonyl oder 2,2,2-Trifluoracetyl stehen, oder
für eine Gruppe der Formel R⁷-CO- stehen,
worin
R⁷ Wasserstoff, Trifluormethyl oder geradkettiges oder verzweigtes Alkoxy mit bis zu 4 und Alkyl mit bis zu 14 Kohlenstoffatomen bedeutet, die gegebenenfalls bis zu 2-fach durch Phenyl, Naphthyl oder Pyridyl substituiert sind, oder
Phenyl oder Naphthyl bedeutet, das gegebenenfalls durch Fluor, Chlor, Trifluormethyl, Trifluormethoxy oder durch geradkettiges oder verzweigtes Alkyl mit bis zu 4 Kohlenstoffatomen substituiert sein kann,
Cyclopropyl, Cyclopentyl, Cyclohexyl, Chinolyl, Chinolyl-N-oxid, Indolyl, Pyridyl, Morpholino oder Piperazinyl bedeutet, oder
einen Rest der Formel 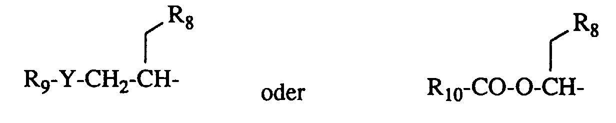 bedeutet,
worin
Y die CO- oder SO₂-Gruppe bedeutet,
R⁸ Phenyl oder Naphthyl bedeutet
R⁹ und R¹⁰ unabhängig voneinander für geradkettiges oder verzweigtes Alkyl mit bis zu 4 Kohlenstoffatomen, Tolyl, Phenyl oder Naphthyl stehen,
R¹⁰ einen Rest der Formel 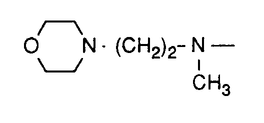 bedeutet,
- R² und R⁴: gleich oder verschieden sind und für Wasserstoff oder geradkettiges oder verzweigtes Alkyl mit bis zu 4 Kohlenstoffatomen stehen,
- R⁵ und R⁶: gleich oder verschieden sind und für Wasserstoff oder für geradkettiges oder verzweigtes Acyl oder Alkoxycarbonyl mit jeweils bis zu 4 Kohlenstoffatomen stehen, oder
für Trimethylsilyl, Triethylsilyl, Triisopropylsilyl, tert.Butyl-dimethylsilyl, Triphenylsilyl, Benzyloxycarbonyl, 4-Nitrobenzyloxycarbonyl, tert.Butoxycarbonyl, Allyloxycarbonyl, 4-Methoxybenzyloxycarbonyl, Formyl, Acetyl oder Trichloracetyl stehen,
und im Fall, daß entweder R⁵ oder R⁶ für Wasserstoff steht,
in Form ihrer Halbacetale und deren Salze.

Außerdem wurde ein Verfahren zur Herstellung der erfindungsgemäßen Verbindungen der allgemeinen Formel (I) gefunden, dadurch gekennzeichnet, daß man
Verbindungen der allgemeinen Formel (III) 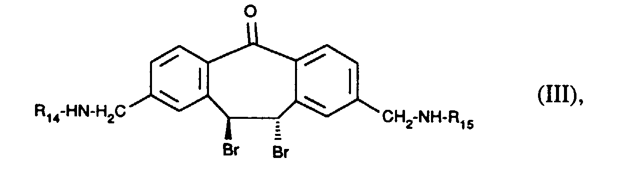 in welcher
- R¹⁴ und R¹⁵: gleich oder verschieden sind und für eine Aminoschutzgruppe, bevorzugt für Phenylmethoxycarbonyl, stehen,
zunächst durch Umsetzungen mit Carbonsäuren der allgemeinen Formel (IV)

R¹⁶-CO₂H (IV),

in welcher
- R¹⁶: den jeweiligen oben aufgeführten Bedeutungsumfang der Substituenten R⁵ und R⁶ mit Ausnahme von Wasserstoff umfaßt,
und in Anwesenheit eines entsprechenden Salzes in die Verbindungen der allgemeinen Formeln (Ia) und (Ib) 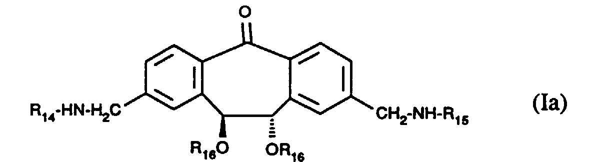 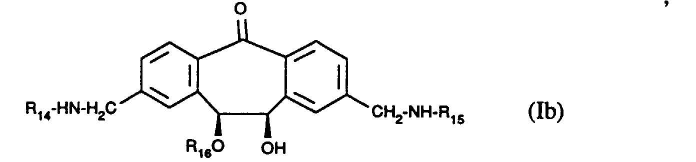 in welcher
- R¹⁴ und R¹⁵: die oben angegebene Bedeutung haben,
- R¹⁶: ebenfalls die oben angegebene Bedeutung hat, aber vorzugsweise für Acetyl steht,
überführt,
im weiteren durch Verseifung die Hydroxyfunktion (R⁵/R^{6 =} H) freisetzt,
und im Fall der oben unter R¹ und R³ noch aufgeführten Substituenten,
zunächst die Reste R¹⁴ und R¹⁵ nach üblichen Methoden, vorzugsweise durch Hydrierung, unter Freisetzung der Aminfunktion abspaltet, und
in einem letzten Schritt mit Verbindungen der allgemeinen Formel (V)

   R¹⁷-CO₂H (V),

   in welcher
R¹⁷ den Bedeutungsumfang der oben aufgeführten Substituenten R¹ und R³ umfaßt,
in inerten Lösemitteln, in Anwesenheit einer Base und/oder Hilfsstoffes, gegebenenfalls unter vorgeschalteter Aktivierung der Carbonsäurefunktion umsetzt.

Das erfindungsgemäße Verfahren kann durch folgendes Formelschema beispielhaft erläutert werden:

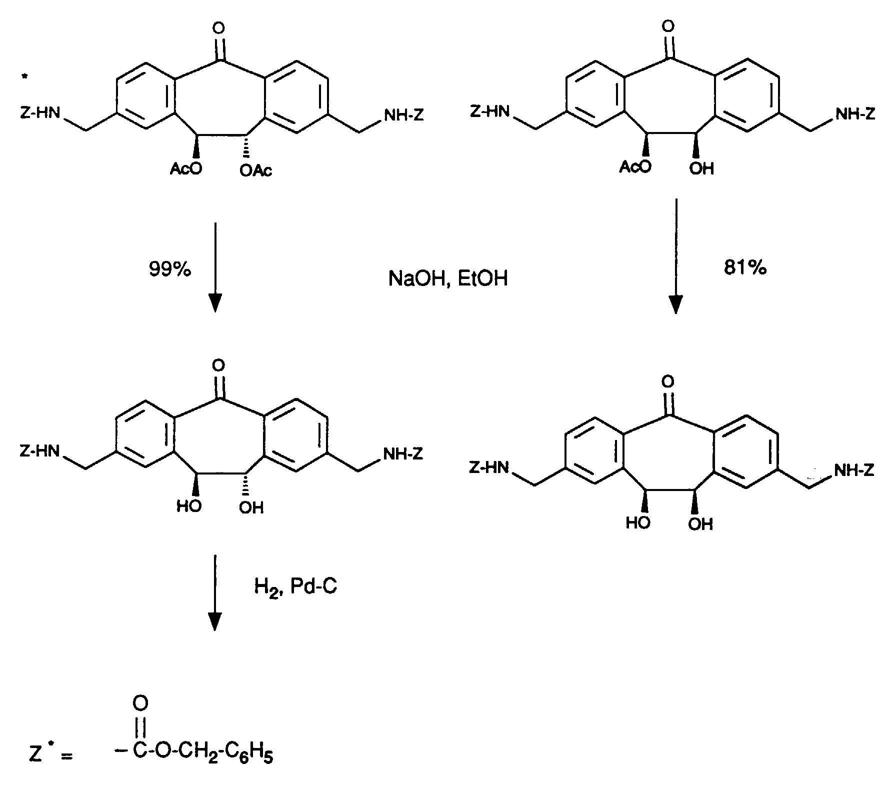

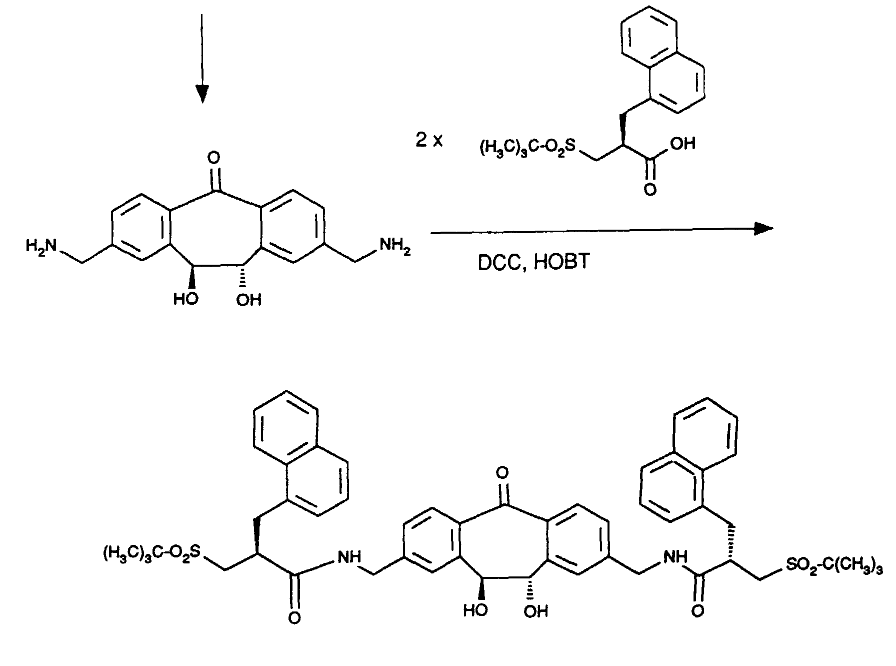

Die Umsetzung mit den Carbonsäuren der allgemeinen Formel (IV) erfolgt im allgemeinen in einem Temperaturbereich von -20°C bis 80°C, vorzugsweise von 0°C bis +40°C und Normaldruck.

Als System Carbonsäure/Salz eignen sich im allgemeinen Essigsäure/Silberacetat, Propionsäure/Silberpropionat, Buttersäure/Silberbutyrat. Bevorzugt ist Essigsäure/Silberacetat.

Als Basen eignen sich für die Verseifung die üblichen anorganischen Basen. Hierzu gehören bevorzugt Alkalihydroxide oder Erdalkalihydroxide wie beispielsweise Natriumhydrid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol, bezogen auf 1 Mol des Esters, eingesetzt. Besonders bevorzugt verwendet man äquimolare Mengen der Reaktanden.

Die Abspaltung der Aminoschutzgruppen (R¹⁴/R¹⁵) erfolgt in an sich bekannter Weise unter sauren oder basischen Bedingungen, oder reduktiv durch katalytische Hydrierung beispielsweise mit Pd/C in organischen Lösemitteln wie Ethern, z.B. Tetrahydrofuran oder Dioxan, oder Alkoholen z.B., Methanol, Ethanol oder Isopropanol.

Die Hydrierung erfolgt im allgemeinen in einem Temperaturbereich von 0°C bis 80°C, vorzugsweise von 0°C bis 40°C.

Im allgemeinen wird die Hydrierung bei erhöhtem Druck von 2 bar bis 8 bar, vorzugsweise von 3 bis 5 bar, durchgeführt.

Als Lösemittel für die Umsetzung mit den Verbindungen der allgemeinen Formel (V) eignen sich die üblichen inerten Lösemittel, die sich unter den Reaktionsbedingungen nicht verändern. Hierzu gehören bevorzugt organische Lösemittel wie Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt sind Dichlormethan, Chloroform, Dimethylformamid oder Tetrahydrofuran.

Als Hilfsstoffe werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'-Diethyl-, N,N'-Diisopropyl-, N,N'-Dicyclohexylcarbodiimid, N-(3-Dimethylaminoisopropyl)-N'-ethyl-carbodiimid-Hydrochlorid, N-Cyclohexyl-N'(2morpholinoethyl)-carbodiimid-metho-p-toluolsulfonat, oder Carbonylverbindungen wie Carbonyldiimidazol, oder 1,2-Oxazoliumverbindungen wie 2-Ethyl-5-phenyl-1,2-oxazolium-3-sulfat oder 2-tert-Butyl-5-methyl-isoxazolium-perchlorat, oder aminoverbindungen wie 2-Ethoxy-1-ethoxycarbonyl-1,2-dihydrochinolin, oder panphosphonsäureanhydrid, oder Isobutylchloroformat, oder Benzotriazolyloxytris(dimethylamino)phosphonium-hexafluorophosphat oder 1-Hydroxybenzotriazol.

Außerdem können beispielsweise Alkalicarbonate, z.B. Natrium- oder Kaliumcarbonat oder -hydrogencarbonat, oder organische Basen wie Trialkylamine, z.B. Triethylamin, Ethyldiisopropylamin, N-Ethylmorpholin, N-Methylpiperidin oder N-Methylmorpholin eingesetzt werden. Bevorzugt ist N-Methylmorpholin.

Die Hilfsstoffe und Basen werden in einer Menge von 1,0 Mol bis 3,0 Mol, bevorzugt 1,0 bis 1,2 Mol, bezogen auf jeweils 1 Mol der Verbindungen der allgemeinen Formel (V), eingesetzt.

Die Reaktionen werden in einem Temperaturbereich von -30°C bis 100°C, vorzugsweise bei 0°C bis 30°C und bei Normaldruck, durchgeführt.

Die Reaktionen können sowohl bei Normaldruck als auch bei erhöhtem oder erniedrigtem Druck (beispielsweise 0,5 bis 5 bar), vorzugsweise bei Normaldruck, durchgeführt werden.

Die Verbindungen der allgemeinen Formeln (IV) und (V) sind bekannt oder können nach üblichen Methoden hergestellt werden.

Die Verbindungen der allgemeinen Formeln (Ia) und (Ib) sind neu und können wie oben beschrieben hergestellt werden.

Die Verbindungen der allgemeinen Formel (III) sind ebenfalls neu und können hergestellt werden, indem man
2,8-Bis-azidomethyl-5-oxo-dibenzo[a,d]cyclohepta-1,4,6-trien der Formel (VI) 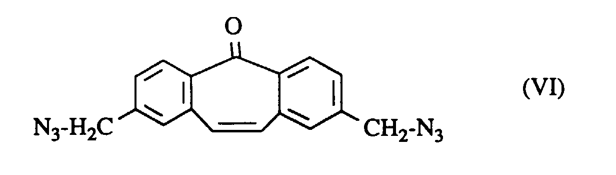 zunächst nach Aufnahme in Ethern, vorzugsweise THF, mit Triphenylphosphan/Wasser unter Ausbildung des Diamins umgesetzt,
und anschließend mit Verbindungen der allgemeinen Formel (VIIa) bzw. (VIIb)

R¹⁸-CO₂H (VIIa),

R¹⁹-CO-Cl (VIIb)

in welcher
R¹⁸ bzw. R¹⁹ den oben aufgeführten Bedeutungsumfang der Reste R¹⁴/R¹⁵ umfaßt,
in inerten Lösemitteln, in Anwesenheit einer Base und Hilfsstoffes, im Falle der Carbonsäuren (VIIa) unter vorgeschalteter Aktivierung der Carbonsäurefunktion, umsetzt,
und in einem letzten Schritt in inerten Lösemitteln eine Bromierung mit elementarem Brom durchführt.

Die Umsetzung mit Triphenylphosphan/H₂O erfolgt in einem Temperaturbereich von 0°C bis +50°C, vorzugsweise bei Raumtemperatur und Normaldruck.

Die Umsetzung mit den Verbindungen der allgemeinen Formel (VII) erfolgt in Analogie zu der oben aufgeführten Beschreibung der Umsetzung mit den Verbindungen der allgemeinen Formel (IV).

Die Bromierung erfolgt in Halogenkohlenwasserstoffen/Eisessig-Gemische, vorzugsweise Dichlormethan/Eisessig und bei Raumtemperatur.

Die Verbindung der Formel (VI) ist ebenfalls neu und kann beispielsweise hergestellt werden, indem man 2,8-Dimethyl-5-oxo-dibenzo[a,d]cyclohepta-1,4,6-trien der Formel (VIII) 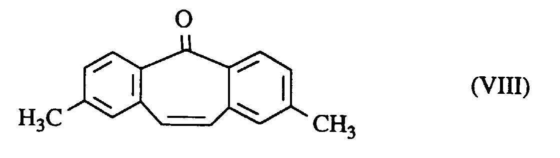 zunächst wie oben beschrieben durch Umsetzung mit N-Bromsuccinimid in einem der oben aufgeführten Lösemittel, vorzugsweise Tetrachlorkohlenstoff, unter Bestrahlung in die Verbindungen der Formel (IX) 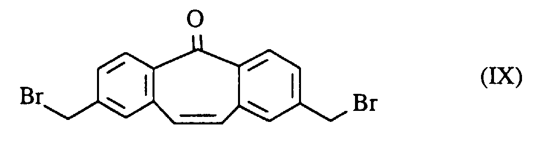 überführt,
und in einem nächsten Schritt mit Lithiumazid in Dimethylsulfoxid umsetzt.

Die Bromierung verläuft in einem Temperaturbereich von +50°C bis +120°C, vorzugsweise von +70°C bis +100°C.

Die Umsetzung mit Lithiumazid wird im allgemeinen in einem Temperaturbereich von 0°C bis +70°C, vorzugsweise bei Raumtemperatur, durchgeführt.

Die Verbindung der Formel (IX) ist neu und wird wie oben beschrieben hergestellt.

Die Verbindung der Formel (VIII) ist neu und kann beispielsweise hergestellt werden, indem man 4-Methyl-2-[2-(3-methylphenyl)ethyl]benzoesäure der Formel (X) 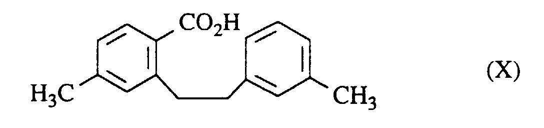 zunächst durch Umsetzung mit Polyphosphorsäure in Sulfolan in die Verbindung der Formel (XI) 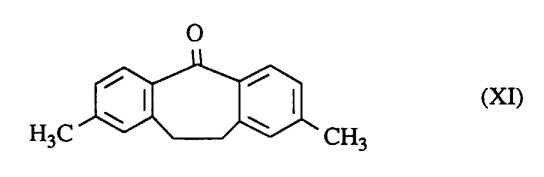 überführt
und in einem nächsten Schritt, wie bereits mehrfach beschrieben, mit N-Bromsuccinimid in Tetrachlorkohlenstoff unter Bestrahlung bromiert und anschließend Bromwasserstoff unter Einwirkung einer Base eliminiert.

Die Umsetzung zu den Verbindungen der Formel (X) verläuft in einem Temperaturbereich von +20°C bis +120°C, vorzugsweise von +60°C bis +100°C für die Bromierung und +20°C bis +60°C für die Eliminierung.

Die Eliminierung findet in einem inerten Lösemittel, bevorzugt Dimethylformamid statt. Bevorzugte Basen sind DBN, DBU und DABCO.

Die Verbindung der Formel (XI) ist neu und kann wie oben beschrieben hergestellt werden.

Die Verbindung der Formel (X) ist ebenfalls neu und wird durch Umsetzung von 2,4-Dimethylbenzoesäure in einer LDA/THF/n-Hexan-Lösung mit 3-Methylbenzylbromid hergestellt.

Die Umsetzung erfolgt in einem Temperaturbereich von -40°C bis +40°C, vorzugsweise von -30°C bis +20°C.

Die hier beschriebenen Inhibitoren sind Inhibitoren der HIV-Protease und sind als solche für alle Zwecke einsetzbar, für die Enzyminhibitoren geeignet sind. Dies ist zum Beispiel der Einsatz in der Diagnostik, um die Präzision und Selektivität von Enzymaktivitätsmessungen zu verbessern. Bei der Affinitätschromatographie können sie als Affinitätslabel dienen und in der Forschung kann man sie zur Aufklärung von Reaktionsmechanismen und der Spezifität enzymatischer Reaktionen verwenden.

Darüber hinaus wurde überraschend gefunden, daß die Verbindungen der allgemeinen Formel (I) eine außerordentlich starke Wirkung gegen Retroviren besitzen. Dies wird mit einem HIV-spezifischen Protease-Enzymtest belegt.

Die Ergebnisse der unten aufgeführten Beispiele wurden nach dem in den folgenden Literaturangaben [vgl. Hansen, J., Billich, S., Schulze, T., Sukrow, S. and Mölling, K. (1988), EMBO Journal, Vol, 7, No. 6, pp. 1785 - 1791] beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert. Die entstandenen Spaltprodukte des synthetischen Peptids wurden über Reverse Phase High Performance Liquid Chromatography (RP-HPLC) analysiert. Die angegebenen IC₅₀-Werte beziehen sich auf die Substanzkonzentration, die unter den oben aufgeführten Testbedingungen eine 50%ige Hemmung der Protease-Aktivität bewirkt.

**Tabelle 1:**

| Bsp.-Nr. | IC₅₀ (µm) |
|---|---|
| 4 | 0,005 |
| 6 | 2 |

Außerdem zeigten die erfindungsgemäßen Verbindungen Wirkung in Lentivirus infizierten Zellkulturen. Dies konnte am Beispiel des HIV-Virus gezeigt werden.

### HIV-Infektion in Zellkultur

Der HIV-Test wurde mit geringen Modifikationen nach der Methode von Pauwels et al. [vgl. Journal of Virological Methods 20, (1988), 309-321] durchgeführt.

Normale menschliche Blutlymphozyten (PBL's) wurden über Ficoll-Hypaque angereichert und im RPMI 1640, 20% fötales Kälberserum mit Phythaemagglutinin (90 µg/ml) und Interleukin-2 (40 U/ml) stimuliert. Zur Infektion mit dem infektiösen HIV wurden PBL's pelletiert und das Zellpellet wurde anschließend in 1 ml HIV-Virusadsorptionslösung suspendiert und 1 Stunde bei 37°C inkubiert.

Die Virusadsorptionslösung wurde zentrifugiert und das infizierte Zellpellet in Wachstumsmedium aufgenommen, so daß 1 x 10⁵ Zellen pro ml eingestellt waren. Die derart infizierten Zellen wurden zu 1 x 10⁴ Zellen/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfsubstanzen in 2er Schritten 2¹⁰fach verdünnt wurden.

Die Testansätze wurden so lange bei 37°C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultierten unter diesen Testbedingungen etwa 20 Syncytien, während die unbehandelte Zellkontrolle keine Syncytien aufwies.

Die IC₅₀-Werte wurden als die Konzentration der erfindungsgemäßen Verbindungen ermittelt, bei der 50% (ca. 10 Syncytien) der virusinduzierten Syncytien bei den behandelten und infizierten Zellen durch die Behandlung mit dieser Verbindung unterdrückt waren.

Es wurde nun gefunden, daß die erfindungsgemäßen Verbindungen HIV infizierte Zellen vor der virusinduzierten Zellzerstörung schützen.

**Tabelle 2**

| Bsp.-Nr. | IC₅₀ (µm) |
|---|---|
| 4 | 10 |
| 5 | 10 |

Die erfindungsgemäßen Verbindungen stellen wertvolle Wirkstoffe zur Behandlung und Prophylaxe von Erkrankungen, hervorgerufen durch Retroviren, in der Human-und Tiermedizin dar.

Als Indikationsgebiete in der Humanmedizin können beispielsweise genannt werden:
1.) Die Behandlung und Prophylaxe von menschlichen Retrovirusinfektionen.
2.) Für die Behandlung oder Prophylaxe von HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.
3.) Für die Behandlung oder die Prophylaxe einer HTLV-I oder HTLV-II Infektion.
4.) Für die Behandlung oder die Prophylaxe des AIDS-carrier Zustandes (AIDS-Überträger-Zustand).

Als Indikationen in der Tiermedizin können beispielsweise angeführt werden:
Infektionen mit
a) Maedivisna (bei Schafen und Ziegen)
b) progressivem Pneumonievirus (PPV) (bei Schafen und Ziegen)
c) caprine arthritis encephalitis Virus (bei Schafen und Ziegen)
d) Zwoegerziekte Virus (bei Schafen)
e) infektiösem Virus der Anämie (des Pferdes)
f) Infektionen verursacht durch das Katzenleukämievirus
g) Infektionen verursacht durch das Virus der Katzen-Immundefizienz (FIV)
h) Infektionen verursacht durch das Virus der Affen-Immundefizienz (SIV)

Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I)/(Ia)/(Ib) enthalten oder die aus einem oder mehreren Wirkstoffen der Formel (I)/(Ia)/(Ib) bestehen, sowie Verfahren zur Herstellung dieser Zubereitungen.

Die Wirkstoffe der Formel (I)/(Ia)/(Ib) sollen in den oben aufgeführten pharmazeutischen Zubereitungen, in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den Verbindungen der Formel (I)/(Ia)/(Ib) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

### Ausgangsverbindungen

### Beispiel I

4-Methyl-2-[2-(3-methylphenyl)ethyl]benzoesäure

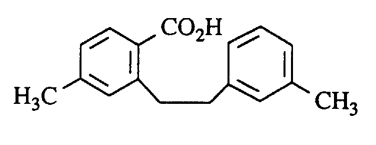

Zu einer Lösung von LDA (0,76 mol) in THF/n-Hexan (1 l/300 ml) wird zwischen 10 - 20°C 2,4-Dimethylbenzoesäure (54 g, 0,36 mol) gelöst in THF (500 ml) zugetropft. Es wird 30 min bei RT nachgerührt und dann auf -20°C gekühlt. Bei dieser Temperatur wird 3-Methylbenzylbromid (80 g, 0,43 mol) in THF (300 ml) zugetropft. Es wird 15 min bei gleicher Temperatur nachgerührt und aufgearbeitet. Hierzu wird zwischen 2 N Salzsäure und EtOAc verteilt, die wäßrige Phase mit EtOAc extrahiert, die organischen Phasen werden vereinigt, mit ges. NaCl gewaschen, getrocknet (MgSO₄) und in vacuo eingeengt. Der Rückstand wird an Kieselgel mit Toluol/EtOAc 10:1 als Eluens gereinigt, und das erhaltene Produkt aus Ether/Petrolether kristallisiert.
Ausbeute: 45,3 g (42% der Theorie)
¹H-NMR (CDCl₃): δ = 2,32 (s, 3H); 2,38 (s, 3H); 2,90 (m, 2H); 3,30 (m, 2H); 7,00 - 7,25 (m, 6H); 8,00 (d, J = 9 Hz, 1H).

### Beispiel II

2,8-Dimethyl-5-oxo-dibenzo[a,d]cyclohepta-1,4-dien

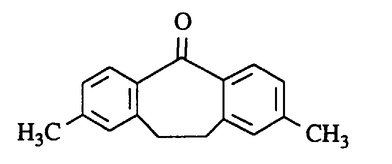

22,65 g (89 mmol) der Verbindung aus Beispiel I wird in Sulfolan (110 g) und Polyphosphorsäure (460 g) 4 h auf 110°C erhitzt. Nach Abkühlen gießt man die Lösung in Wasser (2 l) und extrahiert dreimal mit EtOAc, wäscht die vereinigten organischen Phasen mit Wasser und trocknet sie (MgSO₄). Nach Eindampfen in vacuo wird an Kieselgel mit reinem Toluol als Eluens chromatographiert und das erhaltene Produkt aus Ether / Petrolether kristallisiert.
Ausbeute: 21,05 g (100% d.Th.)
¹H-NMR (CDCl₃): δ = 2,35 (s, 6H), 3,12 (s, 4H); 7,00 (s, 2H); 7,12 (d, J= 8 Hz, 2H); 7,98 (d, J = 8 Hz, 2H).

### Beispiel III

2,8-Dimethyl-5-oxo-dibenzo[a,d]cyclohepta-1,4,6-trien

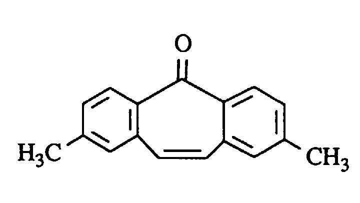

21 g (88,9 mmol) der Verbindung aus Beispiel II wird in Tetrachlormethan (450 ml) gelöst und mit N-Bromsuccinimid (19 g, 107 mmol) bei 55°C mit einer 250 W-Fotolampe bestrahlt. Nach 7 h wird nach Abkühlen der Lösung filtriert, das Filtrat in vacuo eingeengt und der Rückstand in DMF (70 ml) gelöst Die Lösung wird auf 80°C erhitzt und bei dieser Temperatur DBN (13,25 g, 107 mmol) zugegeben. Nach 15 min wird abgekühlt, zwischen Wasser und Ether verteilt, die wäßrige Phase einmal mit Ether extrahiert, und die vereinigten organischen Phasen werden mit 1 N Salzsäure (3x) und Wasser (2x) gewaschen, getrocknet (MgSO₄) und in vacuo eingeengt Der Rückstand wird aus Ether / Petrolether kristallisiert. Man erhält 11,4 g Produkt. Aus der Mutterlauge können durch Chromatographie an Kieselgel mit reinem Toluol als Eluens weitere 2,2 g Produkt gewonnen werden.
Ausbeute: 13,6 g (65% d.Th.)
¹H-NMR (CDCl₃) δ = 2,46 (s, 6H); 6,95 (s, 2H); 7,32 (s, 2H); 7,36 (d, J = 8 Hz, 2H); 8,19 (d, J = 8 Hz, 2H).

### Beispiel IV

2,8-Bis-brommethyl-5-oxo-dibenzo[a,d]cyclohepta-1,4,6-trien

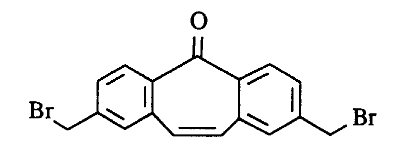

39,8 g (170 mmol) der Verbindung aus Beispiel III und N-Bromsuccinimid werden in Tetrachlormethan (1,5 l) unter Belichtung mit einer 250 W-Fotolampe ca. 4 h unter Rückfluß erhitzt. Die Reaktion wird abgebrochen, wenn im Gemisch von Edukt, Monobromid, Dibromid und Nebenprodukten das gewünsche Dibromid laut DC als Hauptkomponente zu erkennen ist. Man saugt heiß von Ungelöstem ab und wäscht mit Tetrachlormethan nach. Der Rückstand wird aus Dichlormethan umkristallisiert und liefert so 20 g Produkt, verunreinigt mit Succinimid. Das erste Filtrat sowie die Mutterlauge der Kristallisation werden vereinigt, in vacuo eingeengt und der Rückstand an Kieselgel mit reinem Toluol als Eluens chromatographiert. Es werden 12 g Produkt und ca. 17 g Monobromverbindung erhalten. Diese Monobromverbindung (17 g, 54 mmol) wird mit N-Bromsuccinimid (11,6 g, 65 mmol) genau nach obiger Vorschrift umgesetzt und man erhält weitere 8,6 g des gewünschten Dibromids.
Ausbeute: 40,6 g (65,5%)
¹H-NMR (CDCl₃): δ = 4,56 (s, 4H); 7,03 (s, 2H); 7,53 (s, 2H); 7,57 (d, J = 8 Hz, 2H); 8,20 (d, J = 8 Hz, 2H).

### Beispiel V

2,8-Bis-azidomethyl-5-oxo-dibenzo[a,d]cyclohepta-1,4,6-trien

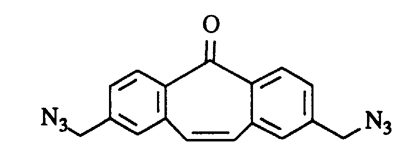

36,5 g (93 mmol) der Verbindung aus Beispiel IV wird in DMSO (250 ml) aufgeschlämmt und dann bei RT mit Lithiumazid (13,7 g, 280 mmol) versetzt. Nach ca. 10 min wird eine vollständige Lösung erhalten. Nach 30 min wird aufgearbeitet. Dazu wird zwischen EtOAc und ges. NaCl verteilt und die wäßrige Phase mit EtOAc extrahiert. Die organischen Phasen werden vereinigt, dreimal mit ges. NaCl gewaschen, getrocknet (MgSO₄) und in vacuo bei RT nicht ganz bis zur Trockne eingeengt. Die verbleibende konzentrierte Lösung wird mit Petrolether versetzt, und der entstehende Feststoff (15,15 g) wird abgesaugt. Die Mutterlauge wird eingeengt und der Rückstand an Kieselgel mit Toluol/EtOAc 10:1 als Eluens chromatographiert. Dies liefert weitere 3,6 g der Titelverbindung.
Ausbeute: 18,75 g (63,5%)
¹H-NMR (CDCl₃): δ = 4,50 (s, 6H); 7,08 (s, 2H); 7,48 (m, 4H); 8,24 (d, J = 8 Hz, 2H).

### Beispiel VI

2,8-Bis-Phenylmethoxycarbonylaminomethyl-5-oxo-dibenzo[a,d]cyclohepta-1,4,6-trien

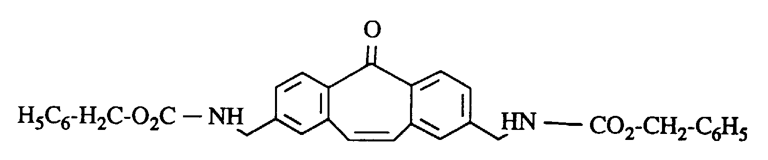

18,75 g (51,1 mmol) der Verbindung aus Beispiel V werden in THF (700 ml) mit Wasser (45 ml) und Triphenylphosphin (29,5 g, 112,3 mmol) versetzt und über Nacht bei RT gerührt. Zur Aufarbeitung wird in vacuo eingeengt, in Dichlormethan aufgenommen, die wäßrige Phase abgetrennt, die organische Phase getrocknet (MgSO₄), in vacuo eingeengt und gründlich im Hochvakuum getrocknet. Das so erhaltene rohe Diamin wird in Dichlormethan gelöst (1 l) und bei -5°C nacheinander mit Triethylamin (33,3 g, 330 mmol) und Benzyloxycarbonylchlorid (37,5 g, 220 mmol) versetzt. Anschließend läßt man auf RT kommen und rührt 3,5 h nach. Zur Aufarbeitung wird mit Dichlormethan verdünnt (1 l), einmal mit Wasser extrahiert, von vorhandenem Feststoff abgesaugt und der Rückstand verworfen. Die wäßrige Phase wird mit Dichlormethan extrahiert, die vereinigten organischen Phasen werden mit gesättigter NaHCO₃ gewaschen, getrocknet (MgSO₄) und in vacuo auf ca. 300 ml eingeengt. Der entstandene geleeartige Feststoff wird abgesaugt, die Mutterlauge vollständig eingeengt und anschließend an Kieselgel mit Toluol/EtOAc 2:1 als Eluens chromatographiert. Die produkthaltigen Fraktionen werden eingeengt und mit dem oben erhaltenen Gelee vereinigt. Dieser Rückstand wird in Dichlormethan / Methanol 1:1 in der Hitze gelöst, mit EtOAc (200 ml) und Toluol (200 ml) versetzt und auf ca. 100 ml in vacuo eingeengt. Dabei fällt gut absaugbarer Feststoff aus, der durch Filtration isoliert wurde.
Ausbeute: 11,5 g (42% d.Th.)
¹H-NMR (DMSO-d₆): δ = 4,39 (d, J = 7 Hz, 4H), 5,00 (s, 4H); 7,10 (s, 2H); 7,30 (m, 10H); 7,45 (d, J = 8 Hz, 2H); 7,51 (s, 2H); 7,91 (t, J = 7 Hz, 2H); 8,03 (d, J = 8 Hz, 2H).

### Beispiel VII

2,8-Bis-phenylmethoxycarbonylaminomethyl-10,11-dibrom-5-oxo-dibenzo[a,d]cyclohepta-1,4-dien

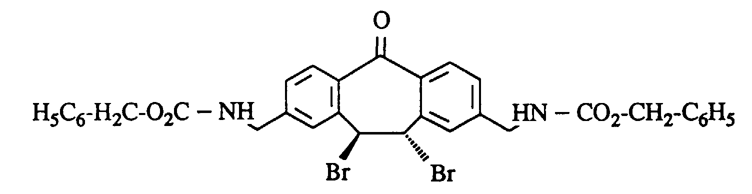

10 g (18,8 mmol) der Verbindung aus Beispiel VI und Brom (6 g, 38 mmol) werden in Dichlormethan (600 ml) und Eisessig (200 ml) unter Lichtausschluß über Nacht gerührt. Dann wird in vacuo eingeengt und der Rückstand an Kieselgel mit Toluol/EtOAc/Ameisensäure 5:1:0,02 chromatographiert.
Ausbeute: 11,0 g (84,5% d.Th.)
¹H-NMR (DMSO-d₆): δ = 4,30 (d, J = 7 Hz, 4H); 5,08 (s, 4H); 6,12 (s, 2H); 7,35 (m, 12H); 7,95 (m, 4H).

### Herstellungsbeispiele

### Beispiel 1, Beispiel 2 und Beispiel 3

(trans)-2,8-Bis-phenylmethoxycarbonylaminomethyl-10,11-diacetoxy-5-oxo-dibenzo[a,d]cyclohepta-1,4-dien

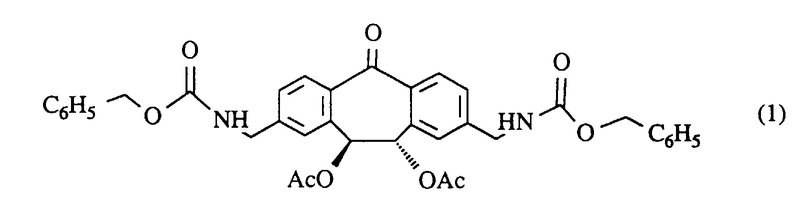

(cis)-10-Acetoxy-2,8-bis-phenylmethoxycarbonylaminomethyl-11-hdroxy-5-oxo-dibenzo[a,d]cyclohepta-1,4-dien

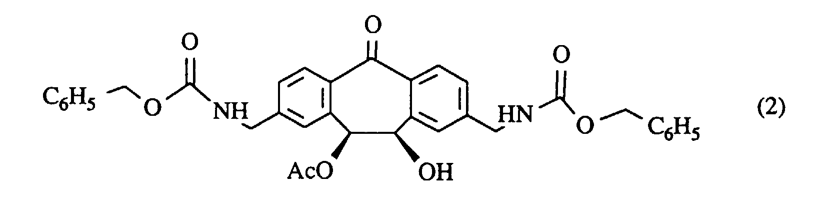

(cis)-11-Acetoxy-5-hydroxy-2,8-bis-phenylmethoxycarbonylaminomethyl-dibenzo[b,e]bicyclo[2.1.3]1-oxaoctan

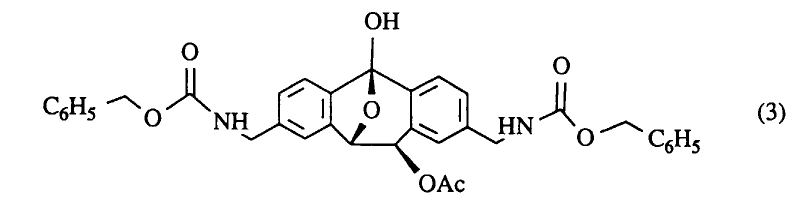

2,44 g (3,53 mmol) der Verbindung aus Beispiel VII und Silberacetat (1,77 g, 10,6 mmol) in trockener Essigsäure (100 ml, hergestellt durch Erhitzen von 150 ml Essigsäure und 10 ml Essigsäureanhydrid unter Rückfluß für 1 h) werden 3 h unter Rückfluß gehalten. Anschließend wird abgekühlt, vom Feststoff abgesaugt und das Filtrat in vacuo eingeengt. Der Rückstand wird an Kieselgel mit Toluol/Essigester 2:1 als Eluens chromatographiert. Dabei eluiert die trans Verbindung (Beispiel 1). Anschließend wird mit Essigester/Aceton 1:1 die cis-Komponente (Beispiel 2) eluiert.

### Beispiel 1

Ausbeute: 1,57 g (68,5% d.Th.)
¹H-NMR (DMSO-d₆): δ = 1,82 (s, 6H); 4,28 (d, J = 7 Hz, 4H); 5,05 (s, 4H); 6,21 (s, 2H); 7,35 (m, 14H), 7,82 (d, J= 8 Hz, 2H); 7,95 (t, J = 7 Hz, 2H):

### Beispiel 2

Ausbeute: 0,43 g (20% d.Th.)
¹H-NMR (DMSO-d₆): δ = 2,02 (s, 3H); 4,29 (d, J = 7 Hz, 4H); 5,06 (m, 5H); 6,12 (s, 1H); 6,19 (d, J = 5 Hz, 1H); 7,31 (m, 13H); 7,50 (s, 1H); 7,73 (d, J = 8 Hz, 1H); 7,79 (d, J = 8 Hz, 1H); 7,96 (t, J = 7 Hz, 2H).

Neben 89% der Verbindung aus Beispiel 2 werden im ¹H-NMR noch 11% der mit Beispiel 2 im Gleichgewicht stehenden Verbindung des Beispiels 3 gesehen. Charakteristische Signale: ¹H-NMR (DMSO-d₆): δ = 4,10 (d, J = 2 Hz, 2H); 4,19 (d, J = 7 Hz, 2H); 5,42 (s, 1H); 7,00 - 7,25 (m, 6H).

### Beispiel 4

(trans)-2,8-Bis-phenylmethoxycarbonylaminomethyl-10,11-dihydroxy-5-oxo-dibenzo[a,d]cyclohepta-1,4-dien

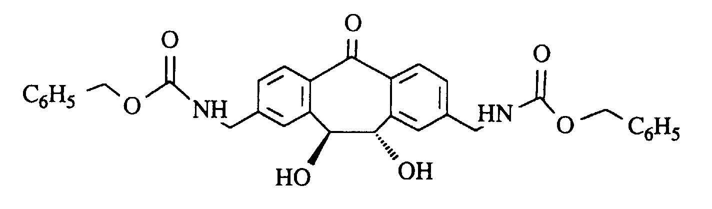

1,8 g (2,76 mmol) der Verbindung aus Beispiel 1 in Ethanol (180 ml) wird mit 0,1 n NaOH (126 ml) versetzt und 1 h bei RT gerührt. Anschließend wird der Alkohol in vacuo abgedampft und die wäßrige Phase dreimal mit EtOAc exträhiert. Die organischen Phasen werden vereinigt, mit ges. NaCl gewaschen, getrocknet (MgSO₄) und in vacuo eingeengt. Der Rückstand wird mit Dichlormethan verrührt und der entstehende Feststoff (1,38 g) abgesaugt. Die Mutterlauge wird eingeengt und an Kieselgel mit Toluol / Essigester 1:1, dann 1:2 als Eluens chromatographiert. Dies ergibt weitere 0,18 g Produkt.
MS (FAB): 567 (M⁺+H, 100%)
¹H-NMR (DMSO-d₆): δ = 4,26 (d, J = 7 Hz, 4H); 4,86 (m, 2H); 5,08 (s, 4H); 5,82 (m, 2H); 7,23 (d, J = 8 Hz, 2H); 7,25 (s, 2H); 7,32 (s, 10H); 7,65 (d, J = 8 Hz, 2H); 7,93 (t, J = 7 Hz, 2H).
Neben 94% der Verbindung aus Beispiel 4 werden im ¹H-NMR noch 6% des isomeren Halbacetals analog zu Beispiel 3 gesehen. Charakteristische Signale: ¹H-NMR (DMSO-d₆): δ = 4,10 (d, J = 7 Hz, 2H); 4,17 (dm, J = 7 Hz, 2H); 5,02 (m, 1H); 5,22 (d, J = 6 Hz, 1H); 5,76 (d, J = 5 Hz, 1H); 7,80 (s, 1H, OH).

### Beispiel 5

(cis-)-2,8-Bis-phenylmethoxycarbonylaminomethyl-10,11-dihydroxy-5-oxo-dibenzo[a,d]cyclohepta-1,4-dien

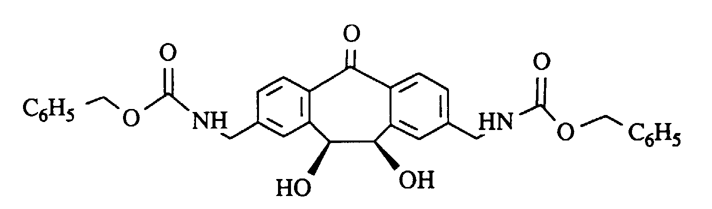

Die Verbindung wird in Analogie zur Vorschrift des Beispiels 4 aus der Verbindung des Beispiels 2 hergestellt.
Ausbeute: 81%
¹H-NMR (DMSO-d₆): δ = 4,28 (d, J = 7 Hz, 4H); 4,89 (d, J = 6 Hz, 2H); 5,07 (s, 4H); 5,70 (d, J = 6 Hz, 2H); 7,23 (d, J = 8 Hz, 2H); 7,32 (s, 10H); 7,50 (s, 2H); 7,73 (d, J = 8 Hz, 2H); 7,94 (t, J = 7 Hz, 2H).
Neben 91% der Verbindung aus Beispiel 4 werden im ¹H-NMR noch 9% des isomeren Halbacetals analog zu Beispiel 3 gesehen. Charakteristische Signale: ¹H-NMR (DMSO-d₆): δ = 4,10 (d, J = 7 Hz, 2H); 4,17 (d, J = 7 Hz, 2H); 5,02 (m, 1H); 5,28 (s, 1H); 7,86 (s, 1H, OH).

### Beispiel 6

(trans)-10,11-Dihydroxy-2,8-bis-[5,5-dimethyl-4,4-dioxo-2-(1-naphthylmethyl)-4-thiahexanoylaminomethyl-5-oxo-dibenzo[a,d]cyclohepta-1,4-dien

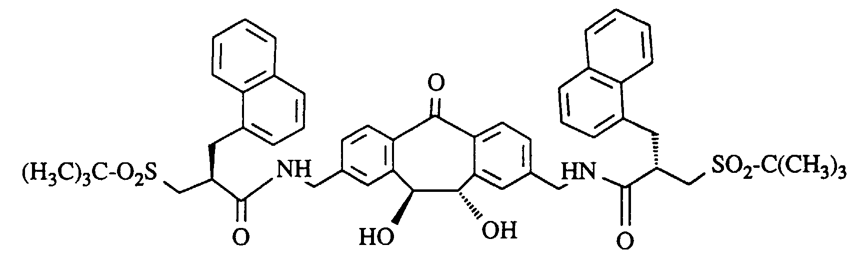

200 mg (0,35 mmol) der Verbindung aus Beispiel 4 in Methanol (100 ml) wird in Gegenwart von 10% Pd/C (40 mg) 3,5 h bei 1 bar hydriert. Anschließend wird vom Katalysator abgesaugt und das Filtrat in vacuo eingeengt. 5,5-Dimethyl-4,4-dioxo-2-(1-naphthylmethyl)-4-thiahexansäure (235 mg, 0,70 mmol) in THF (4 ml) werden unter Eiskühlung mit HOBT (95 mg, 0,70 mmol) und DCC (160 mg, 0,78 mmol) versetzt. Man läßt 1 h bei RT nachrühren und gibt die Reaktionsmischung zu dem rohen oben durch Hydrierung erhaltenen Diamin in Dichlormethan (2 ml). Man rührt 5 h bei RT nach und verdünnt dann mit EtOAc (150 ml). Die Lösung wäscht man mit 4%iger Essigsäure (25 ml), ges. NaHCO₃ und ges. NaCl, trocknet sie (MgSO₄) und engt in vacuo ein. Der Rückstand wird an Kieselgel mit EtOAc/Aceton 5:1, dann 5:2 gereinigt. Man erhält ein geleeartiges Produkt, das zur Kristallisation mit Dichlormethan gründlich durchgearbeitet wird. Aus der Mutterlauge kann nach Lösen in Aceton/Methanol, Versetzen mit Ether und Kristallisation im Kühlschrank weiteres Produkt gewonnen werden.
Ausbeute: 46 mg (14% d.Th.)
¹H-NMR (DMSO-d₆): δ = 1,24 (s, 9H); 3,10 (dd, J = 14 Hz und 3 Hz, 2H); 3,35 (m, 6H); 3,59 (dd, J = 14 Hz und 6 Hz, 2H); 4,16 (dd, J = 16 und 5 Hz, 2H); 4,30 (dd, J = 16 Hz und 5 Hz, 2H), 4,82 (m, 2H); 5,71 (m, 2H); 6,88 (d, J = 8 Hz, 2H); 7,21 (s, 2H); 7,30 - 7,60 (m, 10H), 7,83 (d, J = 8 Hz, 2H); 7,95 (d, J = 8 Hz, 2H); 8,24 (d, J = 7 Hz, 2H); 8,64 (t, J = 5 Hz, 2H).

In Analogie zu der Vorschrift des Beispiels 6 werden die in Tabelle 1 aufgeführten Verbindungen hergestellt:

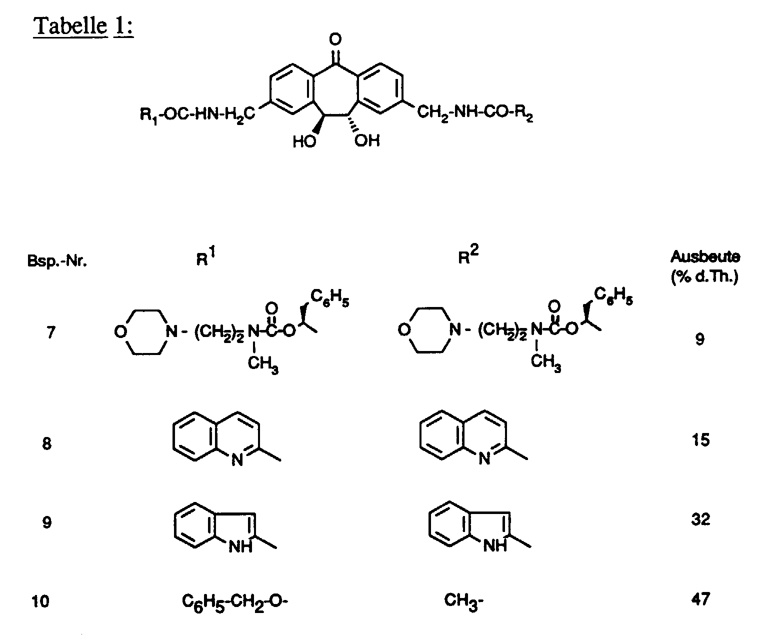

### Beispiel 11

(trans)-10,11-Bis-(tert.butyldimethylsilyloxy)-2,8-bis-phenylmethoxycarbonylaminomethyl-5-oxo-dibenzo[a,d]cyclohepta-1,4-dien

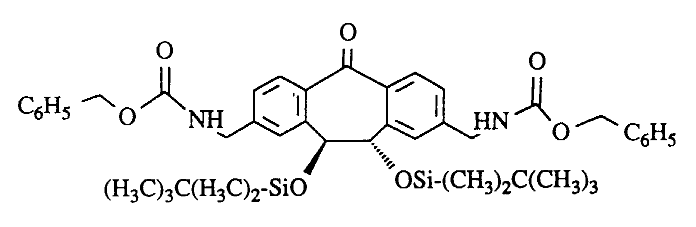

4 g (7,1 mmol) der Verbindung aus Beispiel 4 werden bei RT mit Imidazol (1,9 g, 28,4 mmol) und tert.Butyldimethylsilylchlorid (2,35 g, 15,6 mmol) versetzt und dann 2,5 h bei 50°C gerührt. Zur Aufarbeitung wird mit EtOAc verdünnt, mit Wasser, zweimal mit 1 N Salzsäure und dann mit ges. NaCl gewaschen, getrocknet (MgSO₄) und in vacuo eingeengt. Der Rückstand wird an Kieselgel mit Toluol/EtOAc 10:1 als Eluens gereinigt.
Ausbeute: 5,1 g (90,5% d.Th.)
¹H-NMR (CDCl₃): δ = 0,19 (s, 6H), 0,07 (s, 6H); 0,61 (s, 18H), 4,42 (d, J = 6 Hz, 4H); 4,93 (s, 2H); 5,04 (t, br, J = 6 Hz, 2H); 5,15 (s, 4H); 7,15 (s, 2H); 7,24 (m, 2H); 7,35 (m, 10H); 7,89 (d, J = 8 Hz, 2H).

### Beispiel 12

(trans)-10,11-Dihydroxy-2,8-bis-[methyl(phenylmethoxycarbonyl)amino]methyl-5-oxo-dibenzo [a,d] cyclohepta-1,4,6-trien

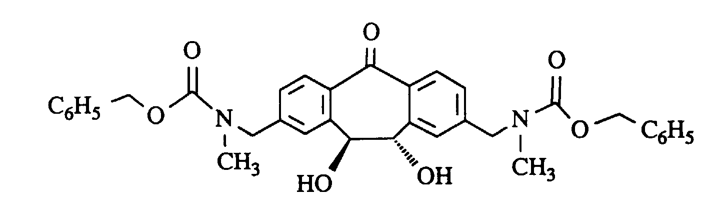

90 mg (0,113 mmol) der Verbindung aus Beispiel 11 werden in DMF (9 ml) unter Eiskühlung mit einer 35%igen Kaliumhydrid-Suspension (11,3 mg, 0,283 mmol) sowie mit Methyliodid (48 mg, 0,34 mmol) versetzt. Nach 15 min bei gleicher Temperatur wird aufgearbeitet. Man verteilt zwischen EtOAc und 1N Salzsäure, extrahiert die wäßrige Phase mit EtOAc, wäscht dann die vereinigten organischen Phasen mit ges. NaCl, trocknet sie (MgSO₄) und engt in vacuo ein. Der Rückstand wird an Kieselgel mit Toluol/EtOAc 10:1 als Eluens gereinigt. Man erhält 48 mg eines Zwischenproduktes, dessen Silylschutzgruppen nach einer allgemeinen Vorschrift zur Silylgruppenabspaltung abgespalten werden (n-Bu₄NF, THF, RT).
Ausbeute: 22 mg (33% d.Th.)
¹H-NMR (CDCl₃): δ = 3,86 und 3,92 (2s, 6H), 4,52 (s, 4H); 4,90 (br, 2H); 5,17 (s, 4H); 7,20 - 7,40 (m, 14H); 7,46 und 7,58 (2br, 2H); 7,84 (d, J = 8 Hz, 2H).

## Patentansprüche

1. 5-Oxo-dibenzo[a,d]cyclohepta-1,4-diene der allgemeinen Formel (I) 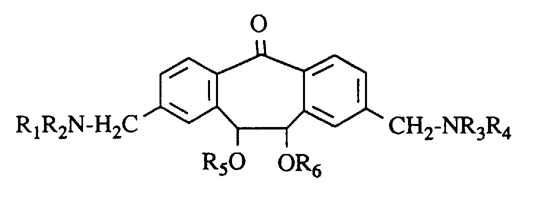 in welcher
R¹ und R³ gleich oder verschieden sind und für eine Aminoschutzgruppe stehen oder
für eine Gruppe der Formel R⁷-CO- stehen,
worin
R⁷ Wasserstoff, Trifluormethyl oder geradkettiges oder verzweigtes Alkoxy mit bis zu 8 Kohlenstoffatomen oder Alkyl mit bis zu 18 Kohlenstoffatomen bedeutet, die gegebenenfalls bis zu 2-fach gleich oder verschieden durch Aryl mit 6 bis 10 Kohlenstoffatomen oder Pyridyl substituiert sind, oder
Aryl mit 6 bis 10 Kohlenstoffatomen bedeutet, das gegebenenfalls durch Halogen, Trifluormethyl, Trifluormethoxy oder durch geradkettiges oder verzweigtes Alkyl mit bis zu 8 Kohlenstoffatomen substituiert ist,
Cycloalkyl mit 3 bis 7 Kohlenstoffatomen bedeutet, oder
Chinolyl, Chinolyl-N-oxid, Indolyl, Pyridyl, Morpholino oder Piperazinyl bedeutet, oder
einen Rest der Formel 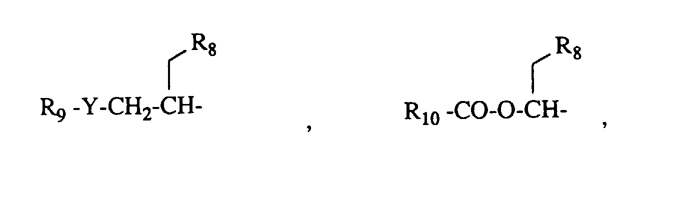 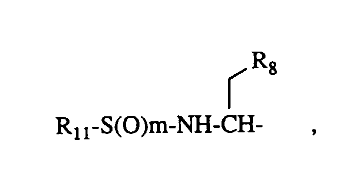
T-NH-(CH₂)ₚ- , 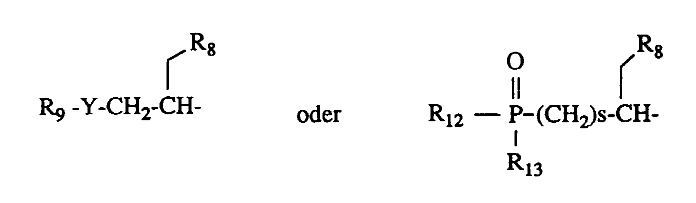
bedeutet, worin
R⁸ Phenyl oder Naphthyl bedeutet,
R⁹, R¹⁰ und R¹¹ unabhängig voneinander geradkettiges oder verzweigtes Alkyl mit bis zu 14 Kohlenstoffatomen bedeuten, das gegebenenfalls durch Phenyl oder Naphthyl substituiert ist, oder Aryl mit 6 bis 10 Kohlenstoffatomen bedeuten, welches seinerseits durch Alkyl mit bis zu 4 Kohlenstoffatomen substituiert ist, oder
R¹⁰ einen Rest der Formel 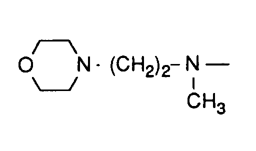 bedeutet,
m eine Zahl 0, 1 oder 2 bedeutet,
T Morpholino oder Cyclohexyl bedeutet,
p eine Zahl 1, 2 oder 3 bedeutet,
Y und Y' unabhängig voneinander für CO- oder SO₂- stehen,
t eine Zahl 0 oder 1 bedeutet,
R¹² und R¹³ unabhängig voneinander für Hydroxy oder Alkoxy mit bis zu 8 Kohlenstoffatomen stehen,
s für eine Zahl 1 oder 2 steht,
R² und R⁴ gleich oder verschieden sind und für Wasserstoff oder geradkettiges oder verzweigtes Alkyl mit bis zu 8 Kohlenstoffatomen stehen,
R⁵ und R⁶ gleich oder verschieden sind und für Wasserstoff oder für geradkettiges oder verzweigtes Acyl oder Alkoxycarbonyl mit jeweils bis zu 8 Kohlenstoffatomen oder für eine Hydroxyschutzgruppe stehen,
und im Fall, daß entweder R⁵ oder R⁶ für Wasserstoff steht,
in Form ihrer Halbacetale und deren Salze.

2. Verbindungen der allgemeinen Formel (I) gemäß Anspruch 1,
in welcher
R¹ und R³ gleich oder verschieden sind und für Benzyloxycarbonyl, 4-Methoxybenzyloxycarbonyl, 4-Nitrobenzyloxycarbonyl, tert.-Butoxycarbonyl, Allyloxycarbonyl, 2-Nitrobenzyloxycarbonyl, Fluorenyl-9-methoxycarbonyl oder 2,2,2-Trifluoracetyl stehen, oder für eine Gruppe der Formel R⁷-CO- stehen,
R⁷ Wasserstoff, Trifluormethyl oder geradkettiges oder verzweigtes Alkoxy mit bis zu 4 Kohlenstoffatomen oder Alkyl mit bis zu 16 Kohlenstoffatomen bedeutet, die gegebenenfalls bis zu 2-fach, gleich oder verschieden, durch Phenyl, Naphthyl oder Pyridyl substituiert sind, oder
Phenyl oder Naphthyl bedeutet, die gegebenenfalls durch Fluor, Chlor, Trifluormethyl, Trifluormethoxy oder durch geradkettiges oder verzweigtes Alkyl mit bis zu 6 Kohlenstoffatomen substituiert sein können,
Cyclopropyl, Cyclopentyl, Cyclohexyl, Chinolyl, Chinolyl-N-oxid, Indolyl, Pyridyl, Morpholino oder Piperazinyl bedeutet, oder
einen Rest der Formel 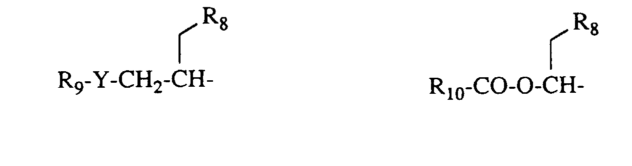 oder 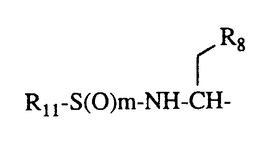 bedeutet,
worin
Y die CO- oder SO₂-Gruppe bedeutet,
R⁸ Phenyl oder Naphthyl bedeutet,
R⁹, R¹⁰ und R¹¹ unabhängig voneinander für geradkettiges oder verzweigtes Alkyl mit bis zu 8 Kohlenstoffatomen, Tolyl, Phenyl oder Naphthyl stehen, oder
R¹⁰ einen Rest der Formel 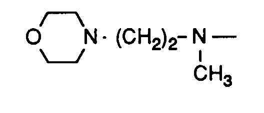 bedeutet,
m eine Zahl 1 oder 2 bedeutet,
R² und R⁴ gleich oder verschieden sind und für Wasserstoff oder geradkettiges oder verzweigtes Alkyl mit bis zu 6 Kohlenstoffatomen stehen,
R⁵ und R⁶ gleich oder verschieden sind und für Wasserstoff oder für geradkettiges oder verzweigtes Acyl oder Alkoxycarbonyl mit jeweils bis zu 6 Kohlenstoffatomen oder
für Trimethylsilyl, Triethylsilyl, Triisopropylsilyl, tert.Butyl-dimethylsilyl, Triphenylsilyl, Benzyloxycarbonyl, 4-Nitrobenzyloxycarbonyl, tert.Butoxycarbonyl, Allyloxycarbonyl, 4-Methoxybenzyloxycarbonyl, Formyl, Acetyl oder Trichloracetyl stehen,
und im Fall, daß entweder R⁵ oder R⁶ für Wasserstoff steht,
in Form ihrer Halbacetale und deren Salze.

3. Verbindungen der allgemeinen Formel (I) gemäß Anspruch 1,
in welcher
R¹ und R³ gleich oder verschieden sind und für Benzyloxycarbonyl, 4-Methoxybenzyloxycarbonyl, 4-Nitrobenzyloxycarbonyl, tert.-Butoxycarbonyl, Allyloxycarbonyl, 2-Nitrobenzyloxycarbonyl, Fluorenyl-9-methoxycarbonyl oder 2,2,2-Trifluoracetyl stehen, oder für eine Gruppe der Formel R⁷-CO- stehen,
worin
R⁷ Wasserstoff, Trifluormethyl oder geradkettiges oder verzweigtes Alkoxy mit bis zu 4 und Alkyl mit bis zu 14 Kohlenstoffatomen bedeutet, die gegebenenfalls bis zu 2-fach durch Phenyl, Naphthyl oder Pyridyl substituiert sind, oder
Phenyl oder Naphthyl bedeutet, das gegebenenfalls durch Fluor, Chlor, Trifluormethyl, Trifluormethoxy oder durch geradkettiges oder verzweigtes Alkyl mit bis zu 4 Kohlenstoffatomen substituiert sein kann,
Cyclopropyl, Cyclopentyl, Cyclohexyl, Chinolyl, Chinolyl-N-oxid, Indolyl, Pyridyl, Morpholino oder Piperazinyl bedeutet, oder
einen Rest der Formel 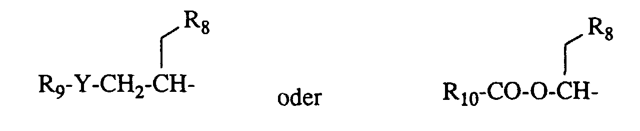 bedeutet,
worin
Y die CO- oder SO₂-Gruppe bedeutet,
R⁸ Phenyl oder Naphthyl bedeutet
R⁹ und R¹⁰ unabhängig voneinander für geradkettiges oder verzweigtes Alkyl mit bis zu 4 Kohlenstoffatomen, Tolyl, Phenyl oder Naphthyl stehen,
R¹⁰ einen Rest der Formel 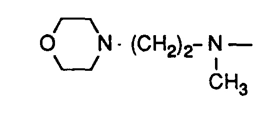 bedeutet,
R² und R⁴ gleich oder verschieden sind und für Wasserstoff oder geradkettiges oder verzweigtes Alkyl mit bis zu 4 Kohlenstoffatomen stehen,
R⁵ und R⁶ gleich oder verschieden sind und für Wasserstoff oder für geradkettiges oder verzweigtes Acyl oder Alkoxycarbonyl mit jeweils bis zu 4 Kohlenstoffatomen stehen, oder
für Trimethylsilyl, Triethylsilyl, Triisopropylsilyl, tert.Butyl-dimethylsilyl, Triphenylsilyl, Benzyloxycarbonyl, 4-Nitrobenzyloxycarbonyl, tert.Butoxycarbonyl, Allyloxycarbonyl, 4-Methoxybenzyloxycarbonyl, Formyl, Acetyl oder Trichloracetyl stehen,
und im Fall, daß entweder R⁵ oder R⁶ für Wasserstoff steht,
in Form ihrer Halbacetale und deren Salze.

4. Verfahren zur Herstellung der Verbindungen der allgemeinen Formel (I) gemäß Anspruch 1, dadurch gekennzeichnet, daß man
Verbindungen der allgemeinen Formel (III) 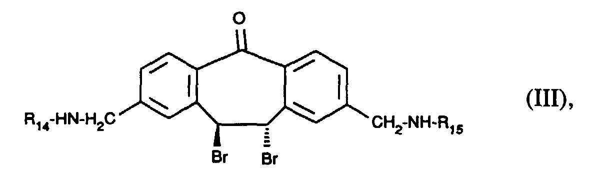 in welcher
R¹⁴ und R¹⁵ gleich oder verschieden sind und für eine Aminoschutzgruppe, bevorzugt für Phenylmethoxycarbonyl, stehen,
zunächst durch Umsetzungen mit Carbonsäuren der allgemeinen Formel (IV)
R¹⁶-CO₂H (IV),
in welcher
R¹⁶ den jeweiligen oben aufgeführten Bedeutungsumfang der Substituenten R⁵ und R⁶ mit Ausnahme von Wasserstoff umfaßt,
und in Anwesenheit eines entsprechenden Salzes in die Verbindungen der allgemeinen Formeln (Ia) und (Ib) 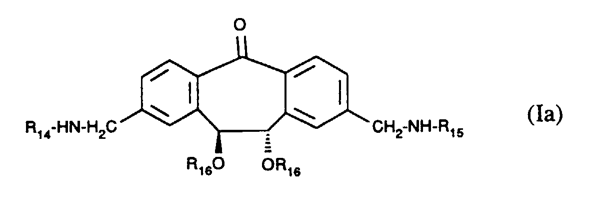 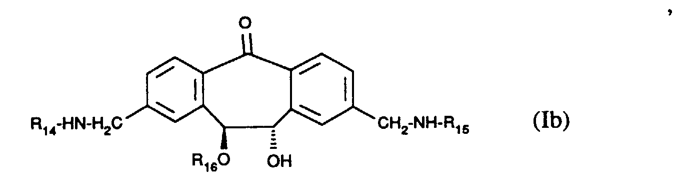 in welcher
R¹⁴ und R¹⁵ die oben angegebene Bedeutung haben,
R¹⁶ ebenfalls die oben angegebene Bedeutung hat, aber vorzugsweise für Acetyl steht,
überführt,
im weiteren durch Verseifung die Hydroxyfunktion (R⁵/R^{6 =} H) freisetzt,
und im Fall der oben unter R¹ und R³ noch aufgeführten Substituenten,
zunächst die Reste R¹⁴ und R¹⁵ nach üblichen Methoden, vorzugsweise durch Hydrierung, unter Freisetzung der Aminfunktion abspaltet, und
in einem letzten Schritt mit Verbindungen der allgemeinen Formel (V)
R¹⁷-CO₂H (V),
in welcher
R¹⁷ den Bedeutungsumfang der oben aufgeführten Substituenten R¹ und R³ umfaßt,
in inerten Lösemitteln, in Anwesenheit einer Base und/oder Hilfsstoffes, gegebenenfalls unter vorgeschalteter Aktivierung der Carbonsäurefunktion umsetzt,.

5. Arzneimittel enthaltend eine oder mehrere der Verbindung gemäß den Ansprüchen 1 bis 3.

## Claims

1. 5-Oxo-dibenzo[a,d]cyc1ohepta-1,4-dienes of the general formula (I) 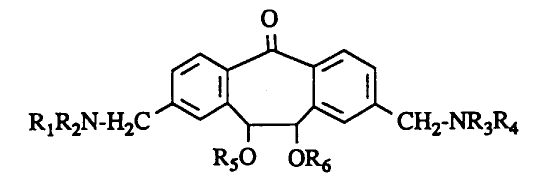 in which
R¹ and R³ are identical or different and represent an amino-protective group or represent a group of the formula R⁷-CO-,
in which
R⁷ denotes hydrogen, trifluoromethyl, or straight-chain or branched alkoxy having up to 8 carbon atoms or alkyl having up to 18 carbon atoms, which are optionally substituted identically or differently up to 2 times by aryl having 6 to 10 carbon atoms or pyridyl, or
denotes aryl having 6 to 10 carbon atoms, which is optionally substituted by halogen, trifluoromethyl, tri-fluoromethoxy, or by straight-chain or branched alkyl having up to 8 carbon atoms,
denotes cycloalkyl having 3 to 7 carbon atoms, or denotes quinolyl, quinolyl N-oxide, indolyl, pyridyl, morpholino or piperazinyl, or
denotes a residue of the formula 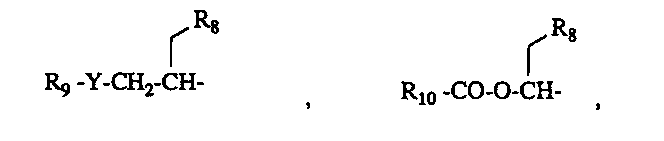 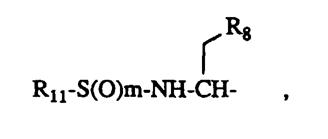
T-NH-(CH₂)ₚ- , 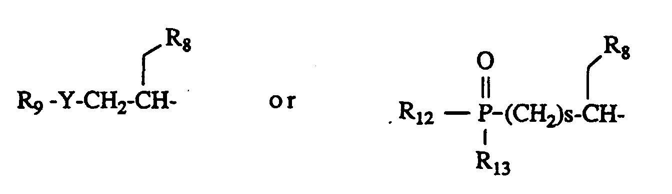
in which
R⁸ denotes phenyl or naphthyl,
R⁹, R¹⁰ and R¹¹, independently of each other, denote straight-chain or branched alkyl having up to 14 carbon atoms, which is optionally substituted by phenyl or naphthyl, or denote aryl having 6 to 10 carbon atoms, which for its part is substituted by alkyl having up to 4 carbon atoms, or
R¹⁰ denotes a residue of the formula 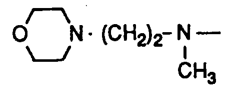
m denotes a number 0, 1 or 2,
T denotes morpholino or cyclohexyl,
p denotes a number 1, 2 or 3,
Y and Y', independently of each other, represent CO- or SO₂-,
t denotes a number 0 or 1,
R¹² and R¹³, independently of each other, represent hydroxyl or alkoxy having up to 8 carbon atoms,
s represents a number 1 or 2,
R² and R⁴ are identical or different and represent hydrogen, or straight-chain or branched alkyl having up to 8 carbon atoms,
R⁵ and R⁶ are identical or different and represent hydrogen, or represent straight-chain or branched acyl or alkoxycarbonyl having in each case up to 8 carbon atoms, or represent a hydroxyl-protective group,
and in the case that either R⁵ or R⁶ represents hydrogen,
in the form of their hemiacetals and salts thereof.

2. Compounds of general formula (I) according to Claim 1,
in which
R¹ and R³ are identical or different and represent benzyloxycarbonyl, 4-methoxybenzyloxycarbonyl, 4-nitrobenzyloxycarbonyl, tert-butoxycarbonyl, allyloxycarbonyl, 2-nitrobenzyloxycarbonyl, fluorenyl-9-methoxycarbonyl or 2,2,2-trifluoroacetyl, or
represent a group of the formula R⁷-CO-,
R⁷ denotes hydrogen, trifluoromethyl, or straight-chain or branched alkoxy having up to 4 carbon atoms or alkyl having up to 16 carbon atoms, which are optionally substituted identically or differently up to 2 times by phenyl, naphthyl or pyridyl, or
denotes phenyl or naphthyl, which can be optionally substituted by fluorine, chlorine, trifluoromethyl, trifluoromethoxy, or by straight-chain or branched alkyl having up to 6 carbon atoms,
denotes cyclopropyl, cyclopentyl, cyclohexyl, quinolyl, quinolyl N-oxide, indolyl, pyridyl, morpholino or piperazinyl, or denotes a residue of the formula 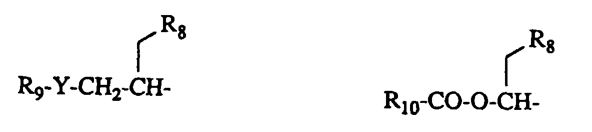 or 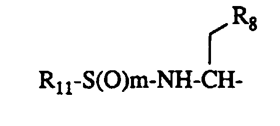 in which
Y denotes the CO or SO₂ group,
R⁸ denotes phenyl or naphthyl,
R⁹, R¹⁰ and R¹¹, independently of each other, represent straight-chain or branched alkyl having up to 8 carbon atoms, tolyl, phenyl or naphthyl, or
R¹⁰ denotes a residue of the formula 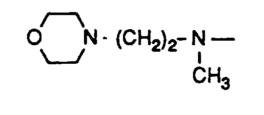
m denotes a number 1 or 2,
R² and R⁴ are identical or different and represent hydrogen, or straight-chain or branched alkyl having up to 6 carbon atoms,
R⁵ and R⁶ are identical or different and represent hydrogen, or represent straight-chain or branched acyl or alkoxycarbonyl having in each case up to 6 carbon atoms or represent trimethylsilyl, triethylsilyl, triisopropylsilyl, tert-butyl-dimethylsilyl, triphenylsilyl, benzyloxycarbonyl, 4-nitrobenzyloxycarbonyl, tert-butoxycarbonyl, allyloxycarbonyl, 4-methoxybenzyloxycarbonyl, formyl, acetyl or trichloroacetyl,
and in the case that either R⁵ or R⁶ represents hydrogen,
in the form of their hemiacetals and salts thereof.

3. Compounds of the general formula (I) according to Claim 1,
in which
R¹ and R³ are identical or different and represent benzyloxycarbonyl, 4-methoxybenzyloxycarbonyl, 4-nitrobenzyloxycarbonyl, tert-butoxycarbonyl, allyloxycarbonyl, 2-nitrobenzyloxycarbonyl, fluorenyl-9-methoxycarbonyl or 2,2,2-trifluoroacetyl, or represent a group of the formula R⁷-CO-
in which
R⁷ denotes hydrogen, trifluoromethyl, or straight-chain or branched alkoxy having up to 4 and alkyl having up to 14 carbon atoms, which are optionally substituted up to 2 times by phenyl, naphthyl or pyridyl, or
denotes phenyl or naphthyl, which can optionally be substituted by fluorine, chlorine, trifluoromethyl, trifluoromethoxy, or by straight-chain or branched alkyl having up to 4 carbon atoms,
denotes cyclopropyl, cyclopentyl, cyclohexyl, quinolyl, quinolyl N-oxide, indolyl, pyridyl, morpholino or piperazinyl, or
denotes a residue of the formula 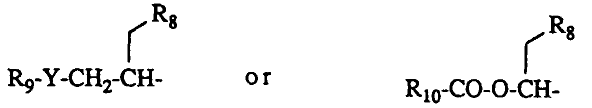 in which
Y denotes the CO or SO₂ group,
R⁸ denotes phenyl or naphthyl
R⁹ and R¹⁰, independently of each other, represent straight-chain or branched alkyl having up to 4 carbon atoms, tolyl, phenyl or naphthyl,
R¹⁰ represents a residue of the formula, 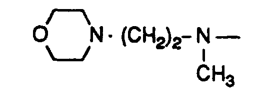
R² and R⁴ are identical or different and represent hydrogen, or straight-chain or branched alkyl having up to 4 carbon atoms,
R⁵ and R⁶ are identical or different and represent hydrogen, or represent straight-chain or branched acyl or alkoxycarbonyl having in each case up to 4 carbon atoms, or
represent trimethylsilyl, triethylsilyl, triisopropylsilyl, tert-butyl-dimethylsilyl, triphenylsilyl, benzyloxycarbonyl, 4-nitrobenzyloxycarbonyl, tert-butoxycarbonyl, allyloxycarbonyl, 4-methoxybenzyloxycarbonyl, formyl, acetyl or trichloroacetyl,
and in the case that either R⁵ or R⁶ represents hydrogen,
in the form of their hemiacetals and salts thereof.

4. Process for preparing the compounds of the general formula (I) according to Claim 1, characterised in that
compounds of the general formula (III) 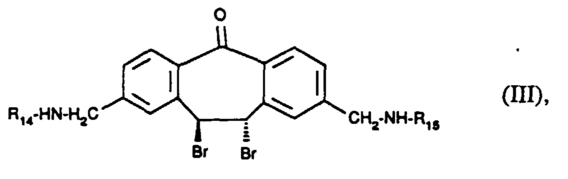 in which
R¹⁴ and R¹⁵ are identical or different and represent an amino-protective group, preferably phenylmethoxycarbonyl,
are first converted by reactions with carboxylic acids of the general formula (IV)
R¹⁶-CO₂H (IV)
in which
R¹⁶ includes the respective range of meaning listed above for the substituents R⁵ and R⁶, with the exception of hydrogen,
and in the presence of a corresponding salt, into the compounds of the general formulae (Ia) and (Ib) 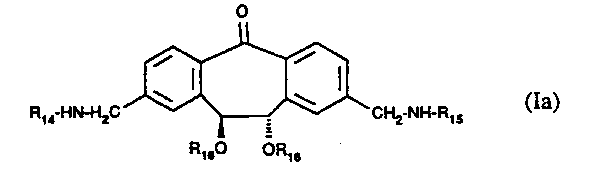 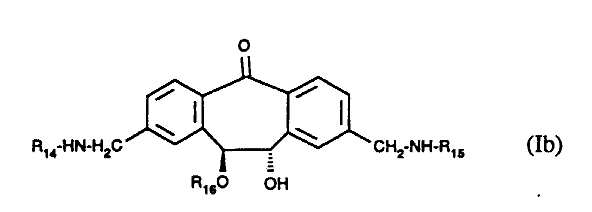 in which
R¹⁴ and R¹⁵ have the abovementioned meaning,
R¹⁶ likewise has the abovementioned meaning, but preferably represents acetyl,
the hydroxyl function (R⁵/R⁶=H) is subsequently liberated by hydrolysis,
and in the case of the remaining substituents listed above under R¹ and R³,
the radicals R¹⁴ and R¹⁵ are first eliminated according to customary methods, preferably by hydrogenation, to liberate the amino function, and
are reacted, in a last step, with compounds of the general formula (V)
R¹⁷-CO₂H (V)
in which
R¹⁷ embraces the range of meaning of the above-listed substituents R¹ and R³,
in inert solvents, in the presence of a base and/or of an auxiliary compound, optionally with prior activation of the carboxylic acid function.

5. Medicaments containing one or more of the compounds according to Claims 1 to 3.

## Revendications

1. 5-oxo-dibenzo[a,d]cyclohepta-1,4-diènes de formule générale (I) 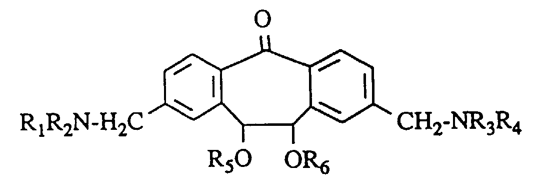 dans laquelle
R¹ et R³ sont identiques ou différents et représentent un groupe protégeant la fonction amino ou représentent
un groupe de formule R⁷-CO-,
dans laquelle
R⁷ est de l'hydrogène, un groupe trifluorométhyle ou un groupe alkoxy linéaire ou ramifié ayant jusqu'à 8 atomes de carbone ou un groupe alkyle linéaire ou ramifié ayant jusqu'à 18 atomes de carbone, qui sont substitués, le cas échéant, jusqu'à 2 fois identiques ou différentes par un radical aryle ayant 6 à 10 atomes de carbone ou un radical pyridyle, ou représente
un groupe aryle ayant 6 à 10 atomes de carbone, qui est substitué, le cas échéant, par un radical halogéno, trifluorométhyle, trifluorométhoxy ou par un radical alkyle linéaire ou ramifié ayant jusqu'à 8 atomes de carbone,
un groupe cycloalkyle ayant 3 à 7 atomes de carbone, ou bien
un groupe quinolyle, quinolyle-N-oxyde, indolyle, pyridyle, morpholino ou pipérazinyle, ou représente
un reste de formule 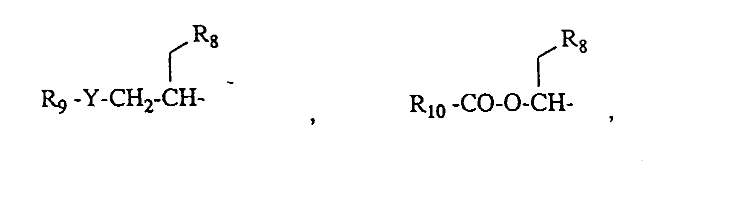 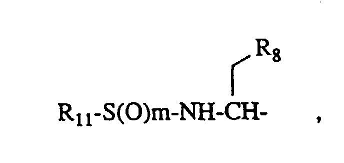
T-NH-(CH₂)ₚ- , 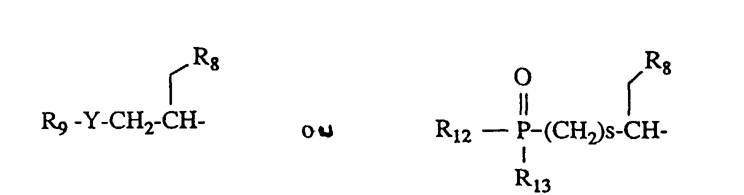
dans laquelle
R⁸ est un groupe phényle ou naphtyle,
R⁹, R¹⁰ et R¹¹ représentent, indépendamment les uns des autres, un groupe alkyle linéaire ou ramifié ayant jusqu'à 14 atomes de carbone, qui est substitué, le cas échéant, par un radical phényle ou naphtyle, ou représentent un groupe aryle ayant 6 à 10 atomes de carbone, qui est substitué de son côté par un radical alkyle ayant jusqu'à 4 atomes de carbone, ou bien
R¹⁰ représente un reste de formule 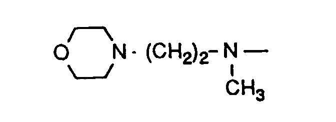
m représente le nombre 0, 1 ou 2,
T est un groupe morpholino ou cyclohexyle,
p est le nombre 1, 2 ou 3,
Y et Y' représentent, indépendamment l'un de l'autre, un groupe CO- ou SO₂-,
t est le nombre 0 ou 1,
R¹² et R¹³ représentent, indépendamment l'un de l'autre, un groupe hydroxy ou un groupe alkoxy ayant jusqu'à 8 atomes de carbone,
s est le nombre 1 ou 2,
R² et R⁴ sont identiques ou différents et représentent de l'hydrogène ou un groupe alkyle linéaire ou ramifié ayant jusqu'à 8 atomes de carbone,
R⁵ et R⁶ sont identiques ou différents et représentent de l'hydrogène ou un groupe acyle ou alkoxycarbonyle linéaire ou ramifié ayant chacun jusqu'à 8 atomes de carbone ou un groupe protégeant la fonction hydroxy,
et au cas où R⁵ ou bien R⁶ représentent de l'hydrogène, sous forme de leurs hémi-acétals et de leurs sels.

2. Composés de formule générale (I) suivant la revendication 1,
dans laquelle
R¹ et R³ sont identiques ou différents et représentent un groupe benzyloxycarbonyle, 4-méthoxybenzyloxycarbonyle, 4-nitrobenzyloxycarbonyle, tertio-butoxycarbonyle, allyloxycarbonyle, 2-nitrobenzyloxycarbonyle, fluorényl-9-méthoxycarbonyle ou 2,2,2-trifluoracétyle, ou représentent un groupe de formule R⁷-CO-,
R⁷ est de l'hydrogène, un groupe trifluorométhyle ou un groupe alkoxy linéaire ou ramifié ayant jusqu'à 4 atomes de carbone ou alkyle linéaire ou ramifié ayant jusqu'à 16 atomes de carbone, qui sont substitués, le cas échéant, jusqu'à 2 fois identiques ou différentes par un groupe phényle, naphtyle ou pyridyle, ou représente
un groupe phényle ou un groupe naphtyle qui peuvent être substitués, le cas échéant, par du fluor, du chlore, un radical trifluorométhyle, trifluorométhoxy ou par un radical alkyle linéaire ou ramifié ayant jusqu'à 6 atomes de carbone,
un groupe cyclopropyle, cyclopentyle, cyclohexyle, quinolyle, quinolyle-N-oxyde, indolyle, pyridyle, morpholino ou pipérazinyle, ou représente
un reste de formule 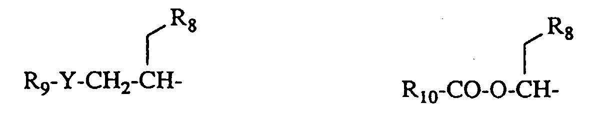 ou 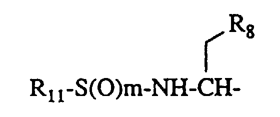 dans laquelle
Y est un groupe CO- ou SO₂-,
R⁸ représente un groupe phényle ou naphtyle,
R⁹, R¹⁰ et R¹¹ représentent, indépendamment les uns des autres, un groupe alkyle linéaire ou ramifié ayant jusqu'à 8 atomes de carbone, tolyle, phényle ou naphtyle, ou bien
R¹⁰ représente un reste de formule 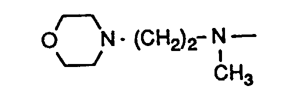
m est le nombre 1 ou 2,
R² et R⁴ sont identiques ou différents et représentent de l'hydrogène ou un groupe alkyle linéaire ou ramifié ayant jusqu'à 6 atomes de carbone,
R⁵ et R⁶ sont identiques ou différents et représentent de l'hydrogène ou un groupe acyle ou alkoxycarbonyle linéaire ou ramifié ayant chacun jusqu'à 6 atomes de carbone, ou représentent un groupe triméthylsilyle, triéthylsilyle, triisopropylsilyle, tertio-butyldiméthylsilyle, triphénylsilyle, benzyloxycarbonyle, 4-nitrobenzyloxycarbonyle, tertio-butoxycarbonyle, allyloxycarbonyle, 4-méthoxybenzyloxycarbonyle, formyle, acétyle ou trichloracétyle,
et au cas où R⁵ ou bien R⁶ représente de l'hydrogène, sous forme de leurs hémi-acétals et de leurs sels.

3. Composés de formule générale (I) suivant la revendication 1,
dans laquelle
R¹ et R³ sont identiques ou différents et représentent un groupe benzyloxycarbonyle, 4-méthoxybenzyloxycarbonyle, 4-nitrobenzyloxycarbonyle, tertio-butoxycarbonyle, allyloxycarbonyle, 2-nitrobenzyloxycarbonyle, fluorényl-9-méthoxycarbonyle ou 2,2,2-trifluoracétyle, ou représentent un groupe de formule R⁷-CO-,
dans laquelle
R⁷ est de l'hydrogène, un groupe trifluorométhyle ou un groupe alkoxy linéaire ou ramifié ayant jusqu'à 4 atomes de carbone et un groupe alkyle linéaire ou ramifié ayant jusqu'à 14 atomes de carbone, qui sont substitués, jusqu'à 2 fois, le cas échéant, par un radical phényle, naphtyle ou pyridyle, ou représente
un groupe phényle ou naphtyle qui peut être substitué, le cas échéant, par du fluor, du chlore, un radical trifluorométhyle, trifluorométhoxy ou par un radical alkyle linéaire ou ramifié ayant jusqu'à 4 atomes de carbone,
un groupe cyclopropyle, cyclopentyle, cyclohexyle, quinolyle, quinolyle-N-oxyde, indolyle, pyridyle, morpholino ou pipérazinyle, ou représente
un reste de formule 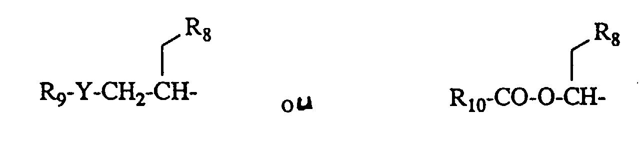 dans laquelle
Y représente le groupe CO- ou SO₂-,
R⁸ est un groupe phényle ou naphtyle,
R⁹ et R¹⁰ représentent, indépendamment l'un de l'autre, un groupe alkyle linéaire ou ramifié ayant jusqu'à 4 atomes de carbone, tolyle, phényle ou naphtyle,
R¹⁰ représente un reste de formule 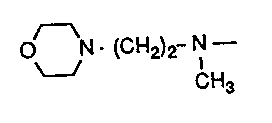
R² et R⁴ sont identiques ou différents et représentent de l'hydrogène ou un groupe alkyle linéaire ou ramifié ayant jusqu'à 4 atomes de carbone,
R⁵ et R⁶ sont identiques ou différents et représentent de l'hydrogène ou un groupe acyle linéaire ou ramifié ou alkoxycarbonyle linéaire ou ramifié ayant chacun jusqu'à 4 atomes de carbone, ou bien
un groupe triméthylsilyle, triéthylsilyle, triisopropylsilyle, tertio-butyldiméthylsilyle, triphénylsilyle, benzyloxycarbonyle, 4-nitro-benzyloxycarbonyle, tertio-butoxycarbonyle, allyloxycarbonyle, 4-méthoxybenzyloxycarbonyle, formyle, acétyle ou trichloracétyle,
et au cas où R⁵ ou bien R⁶ représente de l'hydrogène, sous forme de leurs hémi-acétals et de leurs sels.

4. Procédé de production des composés de formule générale (I) suivant la revendication 1, caractérisé en ce qu'on transforme
des composés de formule générale (III) 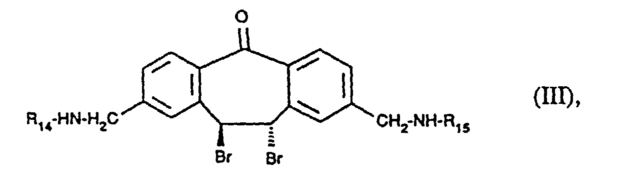 dans laquelle
R¹⁴ et R¹⁵ sont identiques ou différents et représentent un groupe protégeant la fonction amino, de préférence le groupe phénylméthoxycarbonyle,
tout d'abord par réactions avec des acides carboxyliques de formule générale (IV)
R¹⁶-CO₂H (IV),
dans laquelle
R¹⁶ comprend les limites de définitions indiquées ci-dessus pour chacun des substituants R⁵ et R⁶, à l'exception de l'hydrogène,
formules générales (Ia) et (Ib) 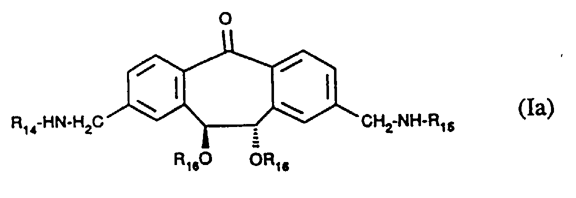 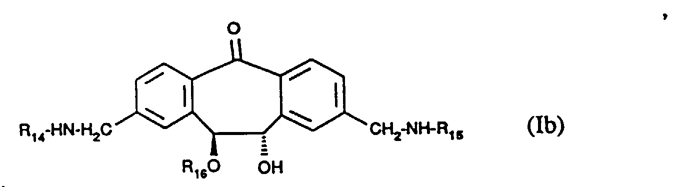 dans lesquelles
R¹⁴ et R¹⁵ ont la définition indiquée ci-dessus,
R¹⁶ a également la définition indiquée ci-dessus, mais représente de préférence le groupe acétyle,
on poursuit en libérant par saponification la fonction hydroxy (R⁵/R⁶=H),
et dans le cas des substituants encore énumérés ci-dessus pour R¹ et R³,
on élimine tout d'abord les restes R¹⁴ et R¹⁵ par des opérations classiques, de préférence par hydrogénation, avec libération de la fonction amine, et
dans une dernière étape, on conduit la réaction avec des composés de formule générale (V)
R¹⁷-CO₂H (V),
dans laquelle
R¹⁷ comprend les limites de définitions des substituants R¹ et R³ indiqués ci-dessus,
dans des solvants inertes, en présence d'une base et/ou d'une substance auxiliaire, le cas échéant avec activation préalable de la fonction acide carboxylique.

5. Médicament contenant un ou plusieurs des composés suivant les revendications 1 à 3.
